(19) **Europäisches Patentamt / European Patent Office / Office européen des brevets**

(11) **EP 4 202 820 B1**

(12) **EUROPEAN PATENT SPECIFICATION**

(45) Date of publication and mention of the grant of the patent:
**31.12.2025 Bulletin 2026/01**

(21) Application number: **21872284.1**

(22) Date of filing: **15.09.2021**

(51) International Patent Classification (IPC):
**G06Q 50/10** (2012.01)    **G06F 16/906** (2019.01)
**A61B 5/00** (2006.01)    **A61B 5/02** (2006.01)
**A61B 5/11** (2006.01)    **A61B 5/16** (2006.01)
**A61B 5/369** (2021.01)    **G06Q 10/10** (2023.01)
**G06Q 10/109** (2023.01)

(52) Cooperative Patent Classification (CPC):
**G06Q 99/00; A61B 5/11; A61B 5/16; A61B 5/352; A61B 5/4035; G06F 18/24137; G06Q 10/10; G06Q 10/109; G06Q 50/10; G06V 40/20; G16H 50/20**

(86) International application number:
**PCT/JP2021/033904**

(87) International publication number:
**WO 2022/065154 (31.03.2022 Gazette 2022/13)**

(54) **INFORMATION PROCESSING DEVICE, INFORMATION PROCESSING METHOD, AND PROGRAM**

INFORMATIONSVERARBEITUNGSVORRICHTUNG, INFORMATIONSVERARBEITUNGSVERFAHREN UND PROGRAMM

DISPOSITIF DE TRAITEMENT D'INFORMATIONS, PROCÉDÉ DE TRAITEMENT D'INFORMATIONS, ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **24.09.2020   JP 2020160161**
**24.09.2020   JP 2020160244**
**24.09.2020   JP 2020160245**
**24.09.2020   JP 2020160246**

(43) Date of publication of application:
**28.06.2023 Bulletin 2023/26**

(73) Proprietor: **JVCKENWOOD Corporation Yokohama-shi, Kanagawa 2210022 (JP)**

(72) Inventors:
• **TAKADA, Motomu**
**Yokohama-shi, Kanagawa 221-0022 (JP)**
• **TSURU, Hideo**
**Yokohama-shi, Kanagawa 221-0022 (JP)**

• **SUWA, Tetsuya**
**Yokohama-shi, Kanagawa 221-0022 (JP)**
• **ODAN, Shohei**
**Yokohama-shi, Kanagawa 221-0022 (JP)**
• **SUGAHARA, Takayuki**
**Yokohama-shi, Kanagawa 221-0022 (JP)**

(74) Representative: **Schmidbauer, Andreas Konrad Wagner & Geyer Partnerschaft mbB Patent- und Rechtsanwälte Gewürzmühlstrasse 5 80538 München (DE)**

(56) References cited:
**WO-A1-2009/070841**    **JP-A- 2010 112 927**
**JP-A- 2011 198 292**    **JP-A- 2019 083 564**
**JP-A- 2019 093 175**    **US-A1- 2019 261 910**
**US-A1- 2019 274 630**

- BRETT ADAMS ET AL: "Sensing and using social context", ACM TRANSACTIONS ON MULTIMEDIA COMPUTING COMMUNICATIONS ANDAPPLICATIONS, ASSOCIATION FOR COMPUTING MACHINERY, US, vol. 5, no. 2, 20 November 2008 (2008-11-20), pages 1 - 27, XP058177869, ISSN: 1551-6857, DOI: 10.1145/1413862.1413864
- DENIS ELBERT ET AL: "An approach for detecting deviations in daily routine for long-term behavior analysis", PERVASIVE COMPUTING TECHNOLOGIES FOR HEALTHCARE (PERVASIVEHEALTH), 2011 5TH INTERNATIONAL CONFERENCE ON, IEEE, 23 May 2011 (2011-05-23), pages 426 - 433, XP032057354, ISBN: 978-1-61284-767-2, DOI: 10.4108/ICST.PERVASIVEHEALTH.2011.246089
- ZEKRI DORSAF ET AL: "Using Learning Techniques to Observe Elderly's Behavior Changes over Time in Smart Home", 23 June 2020, TOPICS IN CRYPTOLOGY - CT-RSA 2020 : THE CRYPTOGRAPHERS' TRACK AT THE RSA CONFERENCE 2020, SAN FRANCISCO, CA, USA, FEBRUARY 24-28, 2020, CORNELL UNIVERSITY LIBRARY, 201 OLIN LIBRARY CORNELL UNIVERSITY ITHACA, NY 14853, PAGE(S) 129 - 141, XP047553296
- OGAWA, SAYAKA; FUJIWARA, KOICHI; YAMAKAWA, TOSHITAKA; ABE, ERIKA; KANO, MANABU: "False Increasing Heart Rate Feedback For Improvement of Game Experience", IPSJ SIG TECHNICAL REPORT. EC, 1 September 2017 (2017-09-01), pages 280 - 286, XP009535601

**Description**

Field

The present disclosure relates to an information processing apparatus, an information processing method, and a program.

Background

A technology for detecting movement of a user and identifying a behavior of the user by using a wearable device that is worn on the user is known.

For example, JP 2003 46630 A describes a mobile phone device that detects acceleration information on a user and controls an operation mode by using the detected acceleration information. JP 2004 184351 A describes an operation information measurement system that measures operation information on a desired part of a user and recognizes a movement state of the whole body.

Brett Adams et. al: "Sensing and using social context" ACM Transactions on multimedia computing communications and applications, association for computing machinery, US, vol. 5, no 2, 20 November 2008, pages 1-27 relates to online algorithms to extract social content based on social rhythms, patterns in time, duration, place and people corresponding to real world activities, thereby formulating social ties from proximity and shared spaces and rhythms. In so doing, the algorithms us a combinatorial clustering process using time, location, duration and people for a rhythm detection. Behavioral rhythms are extracted based on place and time and delineated into rare and frequent and further classified as timed or flexible based on clustering. Furthermore, WO 2009 070 841 A1 describes social multimedia management, and more specifically the organization and sharing of multiple media among multiple users.

Summary

Technical Problem

Here, there is a need to identify a behavior pattern of a user based on information on a behavior state of the user.

An object of the disclosed technology is to provide an information processing apparatus, an information processing method, and a program capable of identifying a behavior pattern of a user based on information on a behavior state of the user.

In accordance with the invention, an information processing apparatus, an information processing method and a program as set forth in the appended claims are provided. An information processing apparatus according to an aspect of the present disclosure inter alia includes: a behavior state detection sensor configured to detect behavior state information on a behavior state of a user; a behavior pattern information generation unit configured to generate behavior pattern information in a multidimensional space formed of coordinate axes based on the behavior state information to generate a group of spaces in each of which density of a behavior pattern information group as a collection of pieces of the behavior pattern information exceeds predetermined density, the coordinate axes representing at least parameters of a date and time, a place, and a duration of detection of the behavior state; a behavior score calculation unit configured to calculate, as a behavior score, information on a size of the space including the behavior pattern information group; and a behavior pattern identification unit configured to identify, as a behavior pattern of the user, the behavior pattern information group in the space for which a value of the behavior score is smaller than a predetermined value.

An information processing method according to an aspect of the present disclosure inter alia includes the steps of: detecting behavior information on a behavior state of a user; generating behavior pattern information in a multidimensional space formed of coordinate axes based on the behavior state information to generate a group of spaces in each of which density of a behavior pattern information group as a collection of pieces of the behavior pattern information exceeds predetermined density, the coordinate axes representing at least parameters of a date and time, a place, and a duration of detection of the behavior state; calculating, as a behavior score, information on a size of the space including the behavior pattern information group; and identifying, as a behavior pattern of the user, the behavior pattern information group in the space for which a value of the behavior score is smaller than a predetermined value.

An information processing method according to an aspect of the present disclosure includes the steps of: detecting behavior state information on a behavior state of a user; detecting biological information related to biological information on the user; calculating an autonomic nerve activity level of the user based on the biological information; and changing intensity of output from an output unit in accordance with intensity of the autonomic nerve activity level.

A program according to an aspect of the present disclosure causes a computer to execute the steps of: detecting behavior state information on a behavior state of a user; generating behavior pattern information in a multidimensional space formed of coordinate axes based on the behavior state information to generate a group of spaces in each of which density of a behavior pattern information group as a collection of pieces of the behavior pattern information exceeds predetermined density, the coordinate axes representing at least parameters of a date and time, a place, and a duration of detection of the behavior state; calculating, as a behavior score, information on a size of the space including the behavior pattern information group; and identifying, as a behavior pattern of the user, the behavior pattern information group in the space for which a value of the behavior score is smaller than a predetermined value.

According to the present disclosure, it is possible to identify a behavior pattern of a user based on information on a behavior state of the user.

Brief Description of Drawings

FIG. 1 is a schematic diagram schematically illustrating an information processing apparatus according to a first embodiment.

FIG. 2 is a block diagram illustrating a configuration example of the information processing apparatus according to the first embodiment.

FIG. 3 is a flowchart illustrating an example of the flow of a process performed by the information processing apparatus according to the first embodiment.

FIG. 4 is a diagram for explaining a multidimensional space for generating behavior pattern information.

FIG. 5 is a diagram for explaining a format for storing a behavior pattern.

FIG. 6 is a flowchart illustrating an example of the flow of a process performed by an information processing apparatus according to a second embodiment.

FIG. 7 is a diagram for explaining an ordinary behavior and an extraordinary behavior.

FIG. 8 is a block diagram illustrating a configuration example of an information processing apparatus according to a third embodiment.

FIG. 9 is a flowchart illustrating an example of the flow of a process performed by the information processing apparatus according to the third embodiment.

FIG. 10 is a graph illustrating one example of a pulse wave.

FIG. 11 is a diagram for explaining a format for storing a behavior pattern.

FIG. 12 is a block diagram illustrating a configuration example of an information processing apparatus according to a fourth embodiment.

FIG. 13 is a diagram for explaining an example of correction data.

FIG. 14 is a flowchart illustrating an example of the flow of a process performed by the information processing apparatus according to the fourth embodiment.

FIG. 15 is a diagram for explaining an example of correction data.

FIG. 16 is a flowchart illustrating an example of the flow of a process performed by an information processing apparatus according to a fifth embodiment.

FIG. 17 is a block diagram illustrating a configuration example of an information processing apparatus according to a sixth embodiment.

FIG. 18A is a diagram for explaining an example of history data of a user.

FIG. 18B is a diagram for explaining an example of the history data of the user.

FIG. 18C is a diagram for explaining an example of the history data of the user.

FIG. 19 is a flowchart illustrating an example of the flow of a learning process according to the sixth embodiment.

FIG. 20 is a flowchart illustrating an example of the flow of a process performed by the information processing apparatus according to the sixth embodiment.

FIG. 21 is a diagram for explaining a configuration example of an information processing system according to a seventh embodiment.

FIG. 22 is a block diagram illustrating a configuration example of a server apparatus according to the seventh embodiment.

FIG. 23 is a flowchart illustrating an example of the flow of a process performed by the server apparatus according to a seventh embodiment.

FIG. 24 is a flowchart illustrating an example of the flow of a learning process according to the seventh embodiment.

FIG. 25 is a flowchart illustrating an example of the flow of a process performed by the information processing apparatus according to the seventh embodiment.

FIG. 26 is a block diagram illustrating a configuration example of an information processing apparatus according to an eighth embodiment.

FIG. 27A is a diagram for explaining a method of displaying a temporal change of an activity level score in a comparative manner.

FIG. 27B is a diagram for explaining the method of displaying a temporal change of an activity level score in a comparative manner.

FIG. 27C is a diagram for explaining the method of displaying a temporal change of an activity level score in a comparative manner.

FIG. 27D is a diagram for explaining the method of displaying a temporal change of an activity level score in a comparative manner.

FIG. 27E is a diagram for explaining the method of displaying a temporal change of an activity level score in a comparative manner.

FIG. 28 is a diagram for explaining a method of outputting information indicating a temporal change of the activity level score.

Description of Embodiments

Embodiments of the present disclosure will be described in detail below with reference to the accompanying drawings. The present disclosure is not limited by the embodiments below, and if a plurality of embodiments are provided, the present disclosure includes configurations that are made by combinations of the embodiments. In addition, in the embodiments below, the same components are denoted by the same reference symbols, and therefore, repeated explanation will be omitted.

First Embodiment

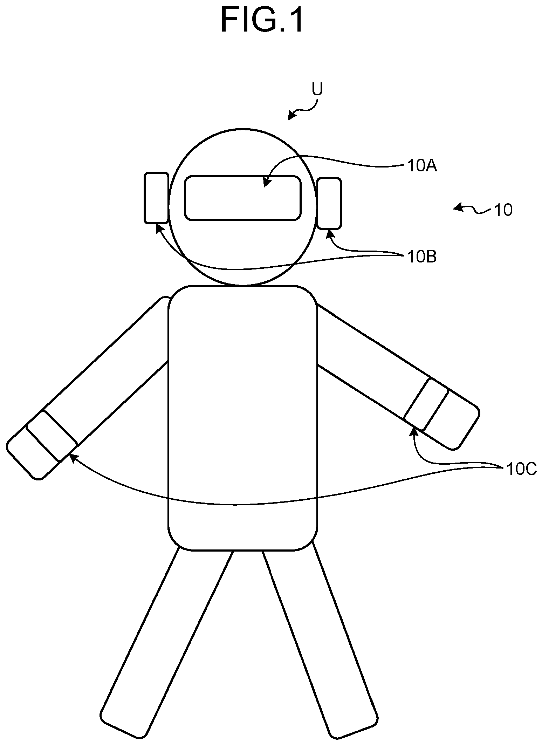

FIG. 1 is a schematic diagram of an information processing apparatus according to a first embodiment. As illustrated in FIG. 1, an information processing apparatus 10 is what is called a wearable device that is worn on a body of a user U. In an example of the present embodiment, the information processing apparatus 10 includes a device 10A that is worn on eyes of the user U, devices 10B that are worn on ears of the user U, and devices 10C that are worn on arms of the user. The device 10A that is worn on the eyes of the user U includes a display unit 26A (to be described later) that outputs visual stimulation (displays an image) to the user U, the devices 10B that are worn on the ears of the user U include sound output units 26B (to be described later) that output auditory stimulation (sound) to the user U, and the devices 10C that are worn on the arms of the user U include tactile stimulation output units 26C (to be described later) that output tactile stimulation to the user U. However, the configuration in FIG. 1 is one example, and the number of the devices and positions of the devices on the user U may be arbitrarily set. For example, the information processing apparatus 10 is not limited to the wearable device, but may be a device, such as what is called a smartphone or a tablet terminal, that is carried by the user U, for example.

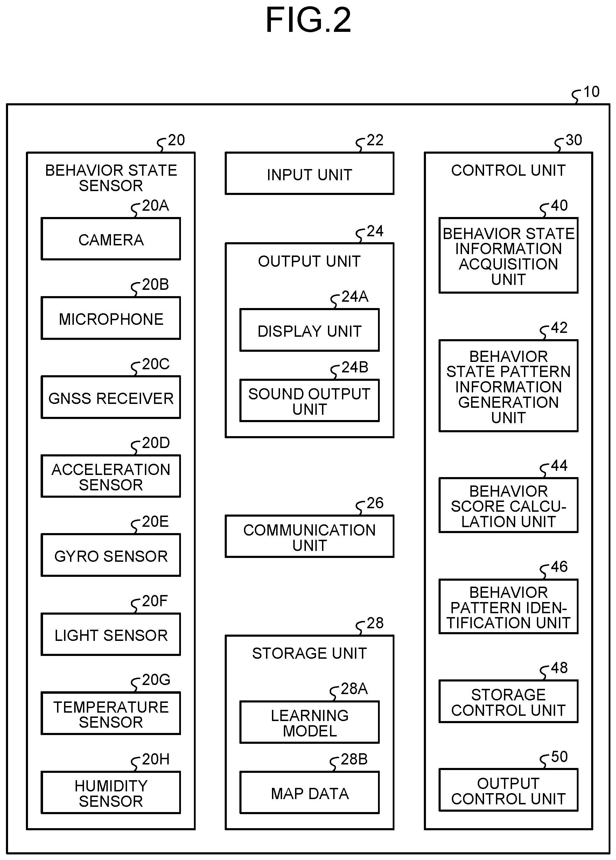

FIG. 2 is a block diagram illustrating a configuration example of the information processing apparatus according to the first embodiment. As illustrated in FIG. 2, the information processing apparatus 10 includes a behavior state sensor 20, an input unit 22, an output unit 24, a communication unit 26, a storage unit 28, and a control unit 30.

The behavior state sensor 20 is a sensor that detects behavior state information on a behavior state of the user U who is wearing the information processing apparatus 10. The behavior state information on the user U may include various kinds of information on a behavior of the user U. The behavior state information on the user U may include information on at least physical body movement of the user U, a date and time at which the behavior is performed, a place where the behavior is performed, and a duration during which the behavior is performed.

The behavior state sensor 20 includes a camera 20A, a microphone 20B, a GNSS receiver 20C, an acceleration sensor 20D, a gyro sensor 20E, a light sensor 20F, a temperature sensor 20G, and a humidity sensor 20H. However, the behavior state sensor 20 may include an arbitrary sensor that detects the behavior state information; for example, the behavior state sensor 20 may include at least one of the camera 20A, the microphone 20B, the GNSS receiver 20C, the acceleration sensor 20D, the gyro sensor 20E, the light sensor 20F, the temperature sensor 20G, and the humidity sensor 20H or may include a different sensor.

The camera 20A is an image capturing apparatus and detects, as the behavior state information, visible light around the information processing apparatus 10 (the user U) and captures an image around the information processing apparatus 10. The camera 20A may be a video camera that captures an image at a predetermined frame rate. A position and an orientation of the camera 20A arranged in the information processing apparatus 10 may be set arbitrarily; for example, the camera 20A may be arranged in the device 10A illustrated in FIG. 1 and an imaging direction may be a direction in which a face of the user U is turned. With this configuration, the camera 20A is able to capture an image of a target object that is looked at by the user U, that is, the target object that is located within a visual field range of the user U. Furthermore, the number of the cameras 20A may be set arbitrarily, and may be singular or plural. Meanwhile, if the plurality of cameras 20A are provided, information on directions in which the cameras 20A are oriented is also acquired.

The microphone 20B is a microphone that detects, as the behavior state information, sound (sound wave information) around the information processing apparatus 10. A position, an orientation, and the number of the microphones 20B arranged in the information processing apparatus 10 may be set arbitrarily. Meanwhile, if the plurality of the microphones 20B are provided, information on directions in which the microphones 20B are oriented is also acquired.

The GNSS receiver 20C is a device that detects, as the behavior state information, positional information on the information processing apparatus 10 (the user U). The positional information in this example is earth coordinates. In the present embodiment, the GNSS receiver 20C is what is called a global navigation satellite system (GNSS) module that receives radio waves from satellites and detects the positional information on the information processing apparatus 10 (the user U).

The acceleration sensor 20D is a sensor that detects, as the behavior state information, acceleration of the information processing apparatus 10 (the user U), and detects, for example, gravity, vibration, and shock.

The gyro sensor 20E is a sensor that detects, as the behavior state information, rotation or an orientation of the information processing apparatus 10 (the user U), and performs detection by using the principle of the Coriolis force, the Euler's force, centrifugal force, or the like.

The light sensor 20F is a sensor that detects, as the behavior state information, light intensity around the information processing apparatus 10 (the user U). The light sensor 20F is able to detect intensity of visible light, infrared light, or ultraviolet light.

The temperature sensor 20G is a sensor that detects, as the behavior state information, temperature around the information processing apparatus 10 (the user U).

The humidity sensor 20H is a sensor that detects, as the behavior state information, humidity around the information processing apparatus 10 (the user U).

The input unit 22 is a device that receives operation performed by the user, and may be, for example, a touch panel or the like.

The output unit 24 outputs an output result obtained by the information processing apparatus 10. The output unit 24 includes, for example, a display unit 24A that displays a video and a sound output unit 24B that outputs sound. In the present embodiment, the display unit 24A is, for example, what is called a head mounted display (HMD). The sound output unit 24B is a speaker that outputs sound.

The communication unit 26 is a module that performs communication with an external apparatus or the like, and may include, for example, an antenna or the like. A communication system of the communication unit 26 is wireless communication in the present embodiment, but an arbitrary communication system is applicable.

The storage unit 28 is a memory for storing various kinds of information, such as contents of calculation performed by the control unit 30 and a program, and includes at least one of a main storage device, such as a random access memory (RAM) or a read only memory (ROM), and an external storage device, such as a hard disk drive (HDD), for example.

The storage unit 28 stores therein a learning model 28A and map data 28B. The learning model 28A is an artificial intelligent (AI) model that is used to identify an environment in which the user U is present, on the basis environmental information. The map data 28B is data including positional information on an existent building or a natural object, and is data in which earth coordinates are associated with an existent building or a natural object. A process using the learning model 28A, the map data 28B, and the like will be described later. Meanwhile, the learning model 28A, the map data 28B, and the program that is stored in the storage unit 28 for the control unit 30 may be stored in a recording medium that is readable by the information processing apparatus 10. Further, the program that is stored in the storage unit 28 for the control unit 30, the learning model 28A, and the map data 28B need not always be stored in the storage unit 28 in advance, but may be acquired by the information processing apparatus 10 from an external apparatus through communication when the data is to be used.

The control unit 30 controls operation of each of the units of the information processing apparatus 10. The control unit 30 is implemented by, for example, causing a central processing unit (CPU), a micro processing unit (MPU), or the like to execute a program that is stored in a storage unit (not illustrated) by using a RAM or the like as a work area. The control unit 30 may be implemented by, for example, an integrated circuit, such as an application specific integrated circuit (ASIC) or a field programmable gate array (FPGA). The control unit 30 may be implemented by a combination of a hardware and software.

The control unit 30 includes a behavior state information acquisition unit 40, a behavior pattern information generation unit 42, a behavior score calculation unit 44, a behavior pattern identification unit 46, a storage control unit 48, and an output control unit 50.

The behavior state information acquisition unit 40 controls the behavior state sensor 20 and causes the behavior state sensor 20 to output the behavior state information on the user U. The behavior state information acquisition unit 40 acquires the behavior state information that is detected by the behavior state sensor 20.

The behavior pattern information generation unit 42 generates behavior pattern information on the basis of the behavior state information that is acquired by the behavior state information acquisition unit 40. The behavior pattern information generation unit 42 generates the behavior pattern information in a multidimensional space, in which at least parameters of a date and time, a place, and a duration of detection of the behavior state of the user U are adopted as coordinate axes, on the basis of the behavior state information, for example.

The behavior score calculation unit 44 calculates a behavior score on the basis of the behavior pattern information that is generated by the behavior pattern information generation unit 42. The behavior score calculation unit 44 generates a group of spaces in each of which density of a behavior pattern information group as a collection of pieces of the behavior state information exceeds predetermined density, for example. The behavior score calculation unit 44 calculates the behavior score of the behavior pattern information group on the basis of the grouped space that includes the behavior pattern information group. Specifically, the behavior score calculation unit 44 calculates, as the behavior score, information on a size of the space that includes the behavior pattern information group, for example.

The behavior pattern identification unit 46 identifies the behavior pattern of the user U on the basis of the behavior score that is calculated by the behavior score calculation unit 44. The behavior pattern identification unit 46 determines, as the behavior pattern of the user U, a behavior that corresponds to the behavior pattern information group for which a value of the behavior score exceeds a predetermined threshold. The behavior pattern identification unit 46 identifies a type of the behavior performed by the user U on the basis of image data, sound data, positional information, acceleration information, posture information, intensity information on infrared light and ultraviolet light, temperature information, humidity information, or the like that is acquired by the behavior state information acquisition unit 40, for example.

The storage control unit 48 causes the storage unit 28 to performs storing. The storage unit 28 stores therein information on the behavior pattern of the user U that is identified by the behavior pattern identification unit 46. The storage control unit 48 stores the information on the behavior pattern of the user U identified by the behavior pattern identification unit 46 in the storage unit 28 in a predetermined format. The predetermined format will be described later.

The output control unit 50 causes the output unit 24 to perform output. The output control unit 50 causes the display unit 24A to display the information on the behavior pattern, for example. The output control unit 50 causes the sound output unit 24B to output the information on the behavior pattern by sound, for example.

Details of process

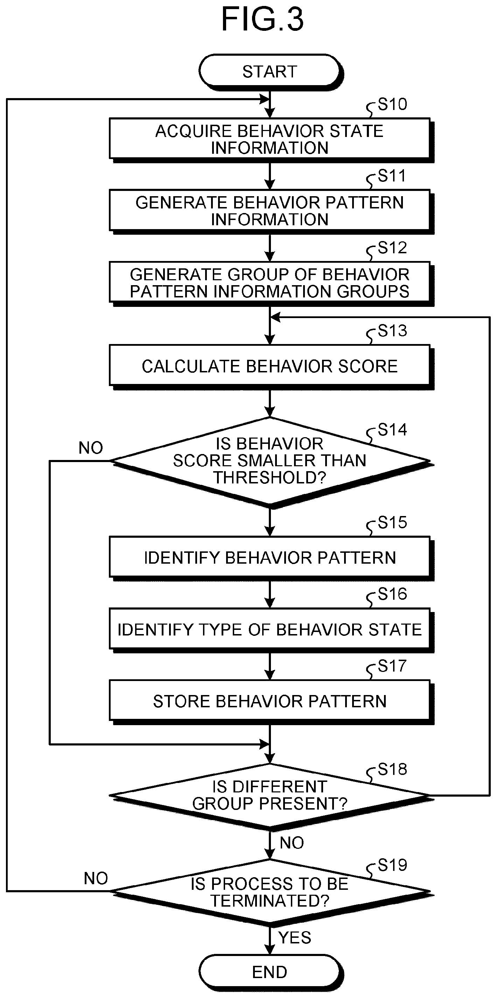

Details of a process performed by the information processing apparatus 10 according to the first embodiment will be descried below with reference to FIG. 3. FIG. 3 is a flowchart illustrating an example of the flow of a process performed by the information processing apparatus 10 according to the first embodiment.

The control unit 30 acquires the behavior state information on the behavior of the user U from the behavior state sensor 20 (Step S10). Specifically, the behavior state information acquisition unit 40 acquires, from the camera 20A, image data that is obtained by capturing an image around the information processing apparatus 10 (the user U). The behavior state information acquisition unit 40 acquires, from the microphone 20B, sound data that is obtained by collecting sound around the information processing apparatus 10 (the user U). The behavior state information acquisition unit 40 acquires, from the GNSS receiver 20C, positional information on the information processing apparatus 10 (the user U). The behavior state information acquisition unit 40 acquires, from the acceleration sensor 20D, acceleration information on the information processing apparatus 10 (the user U). The behavior state information acquisition unit 40 acquires, from the gyro sensor 20E, posture information on the information processing apparatus 10 (the user U). The behavior state information acquisition unit 40 acquires, from the light sensor 20F, intensity information on infrared light and ultraviolet light around the information processing apparatus 10 (the user U). The behavior state information acquisition unit 40 acquires, from the temperature sensor 20G, information on temperature around the information processing apparatus 10 (the user U). The behavior state information acquisition unit 40 acquires, from the humidity sensor 20H, information on humidity around the information processing apparatus 10 (the user U). The behavior state information acquisition unit 40 sequentially acquires pieces of the information as described above for each period. The behavior state information acquisition unit 40 may acquire the pieces of behavior state information at the same timing or different timings. Further, the predetermined period until acquisition of a next piece of behavior state information may be set arbitrarily, and the predetermined period may be the same or different for each piece of environmental information.

The behavior of the user U may include three elements, that is, physical body movement of the user U, a date and time at which the behavior is performed, a place where the behavior is performed, and a duration during which the behavior is performed in the place. The behavior of the user U may include an action, such as "play golf", "watch movie", or "go shopping", in addition to the body movement of the user U. Even when the body movement of the user U acquired by the behavior state information acquisition unit 40 is the same, an action may be different if the positional information is different.

The control unit 30 generates the behavior pattern information on the user U (Step S11). Specifically, the behavior pattern information generation unit 42 generates the behavior pattern information on the user U on the basis of the behavior state information on the user U that is acquired by the behavior state information acquisition unit 40. The control unit 30 generates a group of behavior pattern information groups (Step S12). Specifically, the behavior pattern information generation unit 42 generates a group of spaces in each of which the density of the behavior pattern information group as a collection of pieces of the behavior state information exceeds predetermined density. The control unit 30 calculates the behavior score (Step S13). Specifically, the behavior score calculation unit 44 calculates, as the behavior score, a distance from a center to an end portion of the space that includes the behavior pattern information group. In other words, the behavior score calculation unit 44 calculates, as the behavior score, a size of the space that includes the behavior pattern information group.

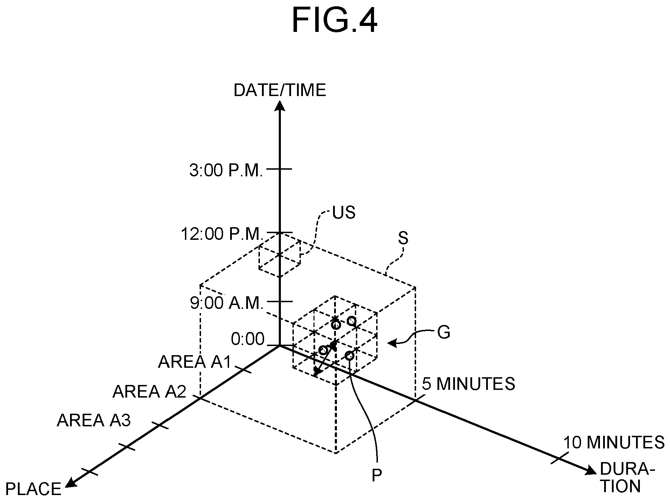

FIG. 4 is a diagram for explaining a multidimensional space for generating the behavior pattern information.

FIG. 4 illustrates a three-dimensional space in which a date and time, a place, and a duration are adopted as the coordinate axes. In FIG. 4, the date and time is from 0:00 to 24:00, the place is a one-dimensional straight line distance from home, and the duration is a duration from when it is determined that the behavior is started to when it is determined that the behavior is terminated, but embodiments are not limited to this example. For example, the place may be a name, an address, or the like.

The behavior pattern information generation unit 42 generates the behavior pattern information by plotting points P at predetermined time intervals in the three-dimensional space illustrated in FIG. 4. For example, the behavior pattern information generation unit 42 plots the points P at an interval of one minute, but embodiments are not limited to this example. For example, the behavior pattern information generation unit 42 may assume that the user U is watching movie for "two hours" "in the vicinity of an area A2" "at around noon". In this case, the behavior pattern information generation unit 42 plots the points P at predetermined time intervals from a certain point, at which "at around noon", "in the vicinity of the area A2", and "two hours" cross one another, until "around 2 o'clock afternoon" such that the points P are arranged parallel to the axis of the date and time. Meanwhile, the behavior pattern identification unit 46 is able to identify that the user U is in a movie theater on the basis of the image data, the sound data, the positional information, the acceleration information, the posture information, the intensity information on infrared light and ultraviolet light, the temperature information, the humidity information, or the like around the user U. If the behavior of the user U is "purchase of groceries", a plurality of candidates for grocery stores, such as a supermarket and an underground shopping area of a department store, may be adopted.

The behavior pattern information generation unit 42 generates, as an identical behavior pattern, a group of spaces in each of which density of the behavior pattern information group that is generated as the behavior pattern information and that is a collection of the points P exceeds predetermined density in the three-dimensional space illustrated in FIG. 4. The behavior pattern information generation unit 42 performs, for example, scanning on a space S for which the behavior pattern information may be generated, by using a unit space US. The behavior pattern information generation unit 42 counts the number of the points P that are included in the unit space US in each of portions of the space S, for example. The behavior pattern information generation unit 42 identifies, as a center of the behavior pattern information, a portion in which the number of the points P included in the unit space US is the largest, for example. The behavior pattern information generation unit 42 counts the number of the points P that are included in the unit spaces US around the unit space US that includes the largest number of the points P, and identifies, as an identical group G, all of the unit spaces US each including about 60% of the points P of the unit space US that includes the largest number of the points P. The behavior score calculation unit 44 calculates, as the behavior score, a length of a perpendicular line that is extended from the center of the group G to any of surfaces, for example. The behavior score calculation unit 44 calculates, as the behavior score, a volume of the group G, for example. Meanwhile, the behavior pattern information generation unit 42 may identify the plurality of groups G in the three-dimensional space illustrated in FIG. 4.

Here, the behavior of the user U may include behaviors of purchases of things that are not frequently purchased, such as "go to a car dealership" for "purchase of a vehicle" and "go to a home improvement store" for "purchase of a chair", for example. The behaviors as described above may be randomly plotted at different places, at different durations, and at different times in the three-dimensional space illustrated in FIG. 4. However, for example, if the user U performs "exercise" for "about 10 minutes" in a nearby "park" at 6 o'clock every morning, points that are plotted on the three-dimensional space are concentrated in the space day by day. The place of the exercise may vary from day to day from several meters to several dozen meters in the same park, and the duration may vary by about 10 minutes to $\pm 2$ minutes. Therefore, the behavior pattern information generation unit 42 may plot and generate pieces of the behavior pattern information with a certain degree of deviation as a relatively large and dense group in the three-dimensional space. In this manner, by selecting the parameters, such as the time, the place, and the duration, which are pieces of information that are relatively simple, as the coordinate axes, it becomes possible to handle the dense group in the three-dimensional space as a single behavior pattern.

In the example illustrated in FIG. 4, the behavior pattern of the user U is provided on the three-dimensional space, but the present disclosure is not limited to this example. In the present embodiment, the behavior pattern of the user U may be generated in an arbitrary multidimensional space. For example, while the place is represented by "the straight line distance from home", the place may be represented by "latitude" and "longitude". In this case, a space in which the behavior pattern information is generated is a four-dimensional space. Furthermore, the date and time is set as "0:00 to 24:00" to represent one day, but a five-dimensional space may be adopted by setting the date and time as "day of week" and adding an axis with a scalar value with seven units. In this case, for example, if the user U works from Monday to Friday and rests on Saturday and Sunday, plots of the behavior pattern information groups may be largely different between a period from Monday to Friday and a period from Saturday and Sunday. By focusing on the axis of the "day of week", it becomes easy to distinguish between a behavior pattern of an ordinary behavior and a behavior pattern of an extraordinary behavior. The behavior patterns of the ordinary behavior and the extraordinary behavior will be described later.

Meanwhile, the duration is set as a duration from start to end of the behavior, but the present disclosure is not limited to this example. For example, as for a behavior as an intermittent event, such as "bat swing is performed 200 times" or "walk (or run) a certain number of steps", the duration may be frequency. For example, when the user U has a habit of regularly performing exercises, and if the duration is changed to frequency, all kinds of behavior patterns can be treated as parameters of "exercise" as "movement", so that it becomes possible to increase the possibility to display data that may be interest the user U.

Referring back to FIG. 3, the control unit 30 determines whether the behavior score is smaller than a threshold (Step S14). Specifically, the behavior pattern identification unit 46 determines whether the behavior score that is calculated by the behavior score calculation unit 44 at Step S13 is smaller than a predetermined threshold. If it is determined that the behavior score is smaller than the threshold (Step S14; Yes), the process goes to Step S15. If it is determined that the behavior score is not smaller than the threshold (Step S14; No), the process goes to Step S18.

If it is determined as Yes at Step S14, the control unit 30 identifies the behavior pattern (Step S15). Specifically, the behavior pattern identification unit 46 identifies, as the behavior pattern of the user U, a behavior that corresponds to the behavior pattern information group for which the behavior score is equal to or smaller than the predetermined threshold.

The control unit 30 identifies a type of a behavior state of the identified behavior pattern (Step S16). Specifically, the behavior pattern identification unit 46 may identify the type of the behavior state performed by the user U, on the basis of the behavior state information that is acquired by the behavior state information acquisition unit 40.

More specifically, the behavior pattern identification unit 46 may identify the behavior state of the user U by using, for example, the learning model 28A. The learning model 28A is an artificial intelligence (AI) model that is constructed by adopting a detection result of the behavior state sensor 20 and information on the type of the behavior state indicated by the detection result of the behavior state sensor 20 as a single data set, and performing learning by using a plurality of data sets as teacher data. The behavior pattern identification unit 46 inputs the detection result of the behavior state sensor 20 to the learned learning model 28A, acquires information indicating the type of the behavior state that is indicated by the detection result, and identifies the type of the behavior state of the user U. The behavior pattern identification unit 46 identifies that, for example, the user U is playing golf, going shopping, or staying in a movie theater by using the learned learning model 28A, for example.

The control unit 30 stores the behavior pattern in the storage unit 28 (Step S17). Specifically, the storage control unit 48 records the behavior pattern identified at Step S16 in a predetermined format.

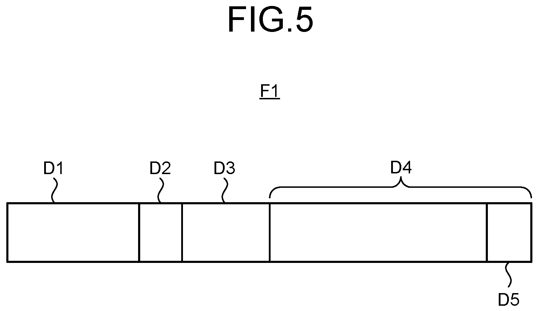

FIG. 5 is a diagram for explaining a format for storing the behavior pattern. In the present embodiment, the behavior pattern may be associated with, in advance, places in which about 16 to 64 kinds of behaviors may be predictable. For example, it is possible to predict "playing golf" from a "golf course", predict "watching movie" from a "movie theater", predict "going shopping" from a "shopping center", and predict "performing exercise" from a "park". In the present embodiment, the positional information on the user U can be acquired from the GNSS receiver 20C of the behavior state sensor 20, so that it is possible to register places where the user U have visited for past several weeks. With this configuration, in the present embodiment, it is possible to assign sequential numbers to candidate behavior patterns related to a life style of the user U.

As illustrated in FIG. 5, a storage format F1 may include a region D1, a region D2, a region D3, a region D4, and a region D5.

In the region D1, a numbering value of the identified behavior pattern is stored. The region D1 is configured with, for example, 3 bytes. In the region D2, the number of dimensions of the space in which the behavior pattern information is plotted as a group is stored. The region D2 is configured with, for example, 1 byte. In this case, a 255-dimensional space may be adopted at maximum. In the region D3, a behavior score R of the behavior pattern information group that is determined as the behavior pattern of the user U is stored. The behavior score R may vary due to a determination error of the behavior pattern, and a smaller value of the behavior score R indicates higher reliability of the behavior pattern. The region D4 is a reserved region. In the region D5 that is a last bit of a region of a last one byte of the reserved region, an identifier that indicates whether the behavior pattern is an ordinary pattern or an extraordinary pattern is stored. In the region D5, 0 is written for the behavior pattern of the ordinary behavior as will be described later, and 1 is written for the behavior pattern of the extraordinary behavior. The reserved region may be used to add incidental information when each of the behavior patterns occurs. The reserved region may have a region of 6 bytes or more for example, and may be used to write each piece of numeral information corresponding to dimensions of N-dimensions (N is an arbitrary integer).

Referring back to FIG. 3, the control unit 30 determines whether a different group is present (Step S18). Specifically, the behavior score calculation unit 44 determines whether a group of behavior pattern information groups for which the behavior score needs to be calculated is present. If it is determined that the different group is present (Step S18; Yes), the process goes to Step S13. If it is determined that the different group is not present (Step S18; No), the process goes to Step S19.

The control unit 30 determines whether the process is to be terminated (Step S19). Specifically, if the control unit 30 receives operation of terminating the process, operation of turning off a power supply, or the like, it is determined that the process is to be terminated. If it is determined that the process is not to be terminated (Step S19; No), the process goes to Step S10. If it is determined that the process is to be terminated (Step S19; Yes), the process in FIG. 3 is terminated.

As described above, the information processing apparatus 10 according to the first embodiment detects the behavior state of the user U and generates the behavior pattern information group corresponding to the behavior state in the multidimensional space. Therefore, the information processing apparatus 10 according to the first embodiment is able to identify the behavior pattern of the user U on the basis of the behavior pattern information group.

Second Embodiment

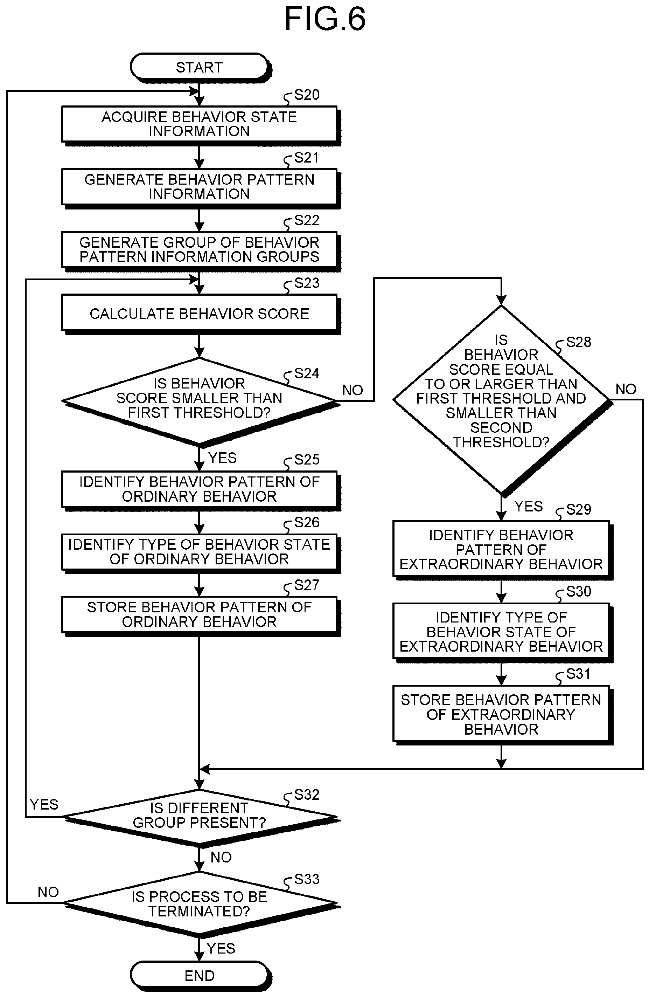

A second embodiment will be described below. FIG. 6 is a flowchart illustrating an example of the flow of a process performed by an information processing apparatus according to the second embodiment. A configuration of the information processing apparatus according to the second embodiment is the same as the configuration of the information processing apparatus 10 illustrated in FIG. 2, and therefore, explanation thereof will be omitted.

In the second embodiment, the information processing apparatus 10 determines whether the identified behavior pattern of the user U is an ordinary behavior that is ordinarily performed or an extraordinary behavior that is extraordinarily performed.

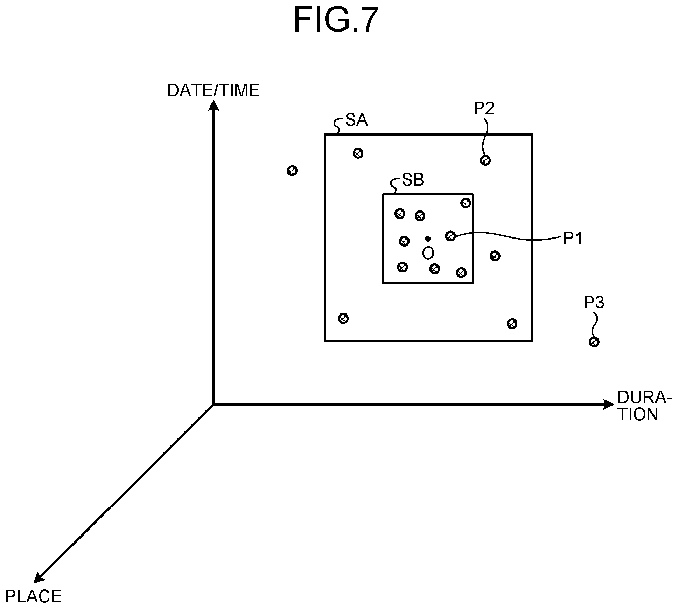

FIG. 7 is a diagram for explaining the ordinary behavior and the extraordinary behavior.

In FIG. 7, a space SA represents a range that is generated as a group of the same behavior pattern, for example. A space SB has the same center as the space SA, and a volume of the space SP is about 60% of a volume of the space SA, for example.

In the second embodiment, the behavior pattern information included in the space SB is a behavior pattern for which the behavior score is smaller than a first threshold. In the second embodiment, the behavior pattern for which the behavior score is smaller than the first threshold is determined as the behavior pattern of the ordinary behavior. In the example illustrated in FIG. 7, a behavior pattern corresponding to a point P1 in the space SB is determined as the behavior pattern of the ordinary behavior.

In the second embodiment, a behavior pattern in a space between the space SA and the space SB is a behavior pattern for which the behavior score is equal to or larger than the first threshold and smaller than a second threshold. In the second embodiment, the behavior pattern for which the behavior score is equal to or larger than the first threshold and smaller than the second threshold is determined as the behavior pattern of the extraordinary behavior. In the example illustrated in FIG. 7, a behavior pattern corresponding to a point P2 in the space between the space SA and the space SB is determined as the behavior pattern of the extraordinary behavior.

In the second embodiment, a behavior pattern included in an outer space of the space SA is a behavior pattern for which the behavior score is equal to or larger than the second threshold. In the second embodiment, the behavior pattern for which the behavior score is equal to or larger than the second threshold is excluded from targets and not included as the behavior pattern of the user.

Referring back to FIG. 6, processes from Step S20 to Step S23 are the same as the processes from Step S10 to Step S13 illustrated in FIG. 3, and therefore, explanation thereof will be omitted.

The control unit 30 determines whether the behavior score is smaller than the first threshold (Step S24). Specifically, the behavior pattern identification unit 46 determines whether the behavior score that is calculated by the behavior score calculation unit 44 at Step S23 is smaller than the predetermined first threshold. If it is determined that the behavior score is smaller than the first threshold (Step S24; Yes), the process goes to Step S25. If it is determined that the behavior score is not smaller than the first threshold (Step S24; No), the process goes to Step S28.

If it is determined as Yes at Step S24, the control unit 30 identifies the behavior pattern of the ordinary behavior (Step S25). Specifically, the behavior pattern identification unit 46 identifies, as the behavior pattern of the ordinary behavior of the user U, a behavior that corresponds to the behavior pattern information group for which the behavior score is smaller than the predetermined first threshold.

The control unit 30 identifies a type of the behavior state of the identified behavior pattern of the ordinary behavior (Step S26). Specifically, the behavior pattern identification unit 46 may identify the type of the behavior state of the ordinary behavior that is performed by the user U, on the basis of the behavior state information that is acquired by the behavior state information acquisition unit 40.

The control unit 30 stores the behavior pattern of the ordinary behavior in the storage unit 28 (Step S27). Specifically, the storage control unit 48 stores the behavior pattern of the ordinary behavior that is identified at Step S25 in a predetermined format.

If it is determined as No at Step S24, the control unit 30 determines whether the behavior score is equal to or larger than the first threshold and smaller than the second threshold (Step S28). Specifically, the behavior pattern identification unit 46 determines whether the behavior score that is calculated by the behavior score calculation unit 44 at Step S23 is equal to or larger than the predetermined first threshold and smaller than the second threshold. If it is determined that the behavior score is equal to or larger than the first threshold and smaller than the second threshold (Step S28; Yes), the process goes to Step S29. If it is determined that the behavior score is not equal to or larger than the first threshold and smaller than the second threshold (Step S28; No), the process goes to Step S32.

If it is determined as Yes at Step S28, the control unit 30 identifies the behavior pattern of the extraordinary behavior (Step S29). Specifically, the behavior pattern identification unit 46 identifies, as the behavior pattern of the extraordinary behavior, a behavior that corresponds to the behavior pattern information group for which the behavior score is equal to or larger than the predetermined first threshold and smaller than the second threshold.

The control unit 30 identifies a type of the behavior state of the identified behavior pattern of the extraordinary behavior (Step S30). Specifically, the behavior pattern identification unit 46 may identify the type of the behavior state of the extraordinary behavior that is performed by the user U, on the basis of the behavior state information that is acquired by the behavior state information acquisition unit 40.

The control unit 30 stores the behavior pattern of the ordinary behavior in the storage unit 28 (Step S31). Specifically, the storage control unit 48 stores the behavior pattern of the extraordinary behavior that is identified at Step S25 in a predetermined format.

Processes at Step S32 and Step S33 are the same as the processes at Step S18 and Step S19 illustrated in FIG. 3, and therefore, explanation thereof will be omitted.

As described above, the information processing apparatus 10 according to the second embodiment determines whether the behavior pattern is a behavior pattern of the ordinary behavior or the extraordinary behavior on the basis of the behavior score. With this configuration, the information processing apparatus 10 according to the second embodiment is able to determine whether the same behavior is an ordinary routine or a new behavior.

Specifically, identification of the ordinary behavior and the extraordinary behavior in the second embodiment may be adopted to determine whether a user has an interest or not, in particular, whether a behavior pattern that occurs in a commuting time on a weekday is a routine behavior or an intentionally performed behavior pattern. For example, when a user goes to work from a certain station to a different station at a fixed time every day, it is possible to determine that a behavior of walking to the station is a routine rather than an active behavior that is performed with an interest if the behavior is performed at the same time, and therefore, it is possible to perform calculation while statistically eliminating data of this behavior pattern.

Third Embodiment

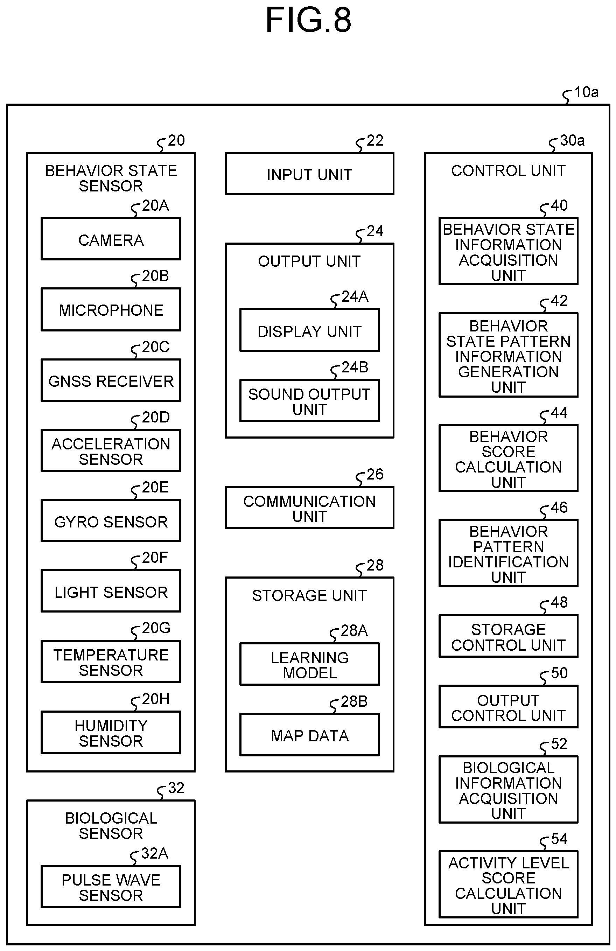

An information processing apparatus according to a third embodiment will be described below with reference to FIG. 8. FIG. 8 is a block diagram illustrating a configuration example of the information processing apparatus according to the third embodiment.

As illustrated in FIG. 8, an information processing apparatus 10a is different from the information processing apparatus 10 illustrated in FIG. 2 in that a biological sensor 32 is provided and a control unit 30a includes a biological information acquisition unit 52 and an activity level score calculation unit 54.

The behavior of the user U includes biological information, such as a degree of excitement, in addition to the physical movement. Therefore, when the behavior pattern of the user U is to be identified, it is preferable to take into account a psychological situation of the user U at the time of the behavior. The information processing apparatus 10a calculates an activity level score that indicates a degree at which the user U enjoys the behavior.

The biological sensor 32 is a sensor that detects biological information on the user U. The biological sensor 32 may be arranged at an arbitrary position as long as it is possible to detect the biological information on the user U. It is preferable that the biological information used here is information for which a value changes depending on the state of the user U, instead of stable information, such as a fingerprint, for example. Furthermore, it is further preferable that the biological information used here is information on an autonomic nerve of the user U, that is, information for which a value changes regardless of intention of the user U. Specifically, the biological sensor 32 includes a pulse wave sensor 32A and detects, as the biological information, a pulse wave of the user U. The biological sensor 32 may include a brain wave sensor that detects a brain wave of the user U.

The pulse wave sensor 32A is a sensor that detects a pulse wave of the user U. The pulse wave sensor 32A may be a transmissive photoelectric sensor that includes a light emitting unit and a light receiving unit, for example. In this case, for example, the pulse wave sensor 32A may be configured such that the light emitting unit and the light receiving unit face each other across a fingertip of the user U and the light receiving unit receives light that has transmitted through the fingertip, and measure a waveform of a pulse by using the fact that a blood flow increases with an increase in pulse wave pressure. However, the pulse wave sensor 32A is not limited to the example as described above, any type that is able to detect a pulse wave is applicable.

The biological information acquisition unit 52 controls the biological sensor 32 and causes the biological sensor 32 to detect the biological information. The biological information acquisition unit 52 acquires the biological information that is detected by the biological sensor 32.

The activity level score calculation unit 54 calculates an autonomic nerve activity level on the basis of the biological information that is acquired by the biological information. A method of calculating the autonomic nerve activity level will be described later. The activity level score calculation unit 54 calculates the activity level score. The activity level score calculation unit 54 calculates an activity level score on the basis of the behavior score that is calculated by the behavior score calculation unit 44, the behavior pattern that is identified by the behavior pattern identification unit 46, and the autonomic nerve activity level.

Details of process

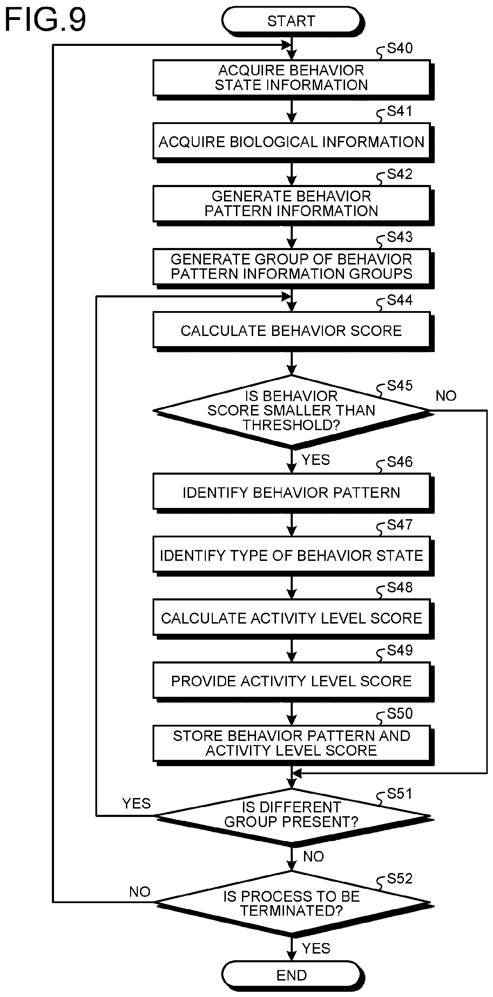

Details of a process performed by the information processing apparatus 10a according to the third embodiment will be described below with reference to FIG. 9. FIG. 9 is a flowchart illustrating an example of the flow of a process performed by the information processing apparatus 10a according to the third embodiment.

A process at Step S40 is the same as the process at Step S10 illustrated in FIG. 3, and therefore, explanation thereof will be omitted.

The control unit 30a acquires the biological information on the user U (Step S41). Specifically, the biological information acquisition unit 52 controls the pulse wave sensor 32A of the biological sensor 32 and acquires pulse wave information on the user U. In the present embodiment, as will be described later, the autonomic nerve activity level, which is an index that indicates a degree of stress, a degree of relaxation, a degree of interest, and a degree of concentration in a psychological state of the user U, is calculated by using the pulse wave information on the user U.

Processes from Step S42 to Step S47 are the same as the processes from Step S12 to Step S17 illustrated in FIG. 3, and therefore, explanation thereof will be omitted.

The control unit 30a calculates the activity level score (Step S48). Specifically, the activity level score calculation unit 54 calculates the activity level score of the user U on the basis of the pulse wave information that is acquired at Step S41.

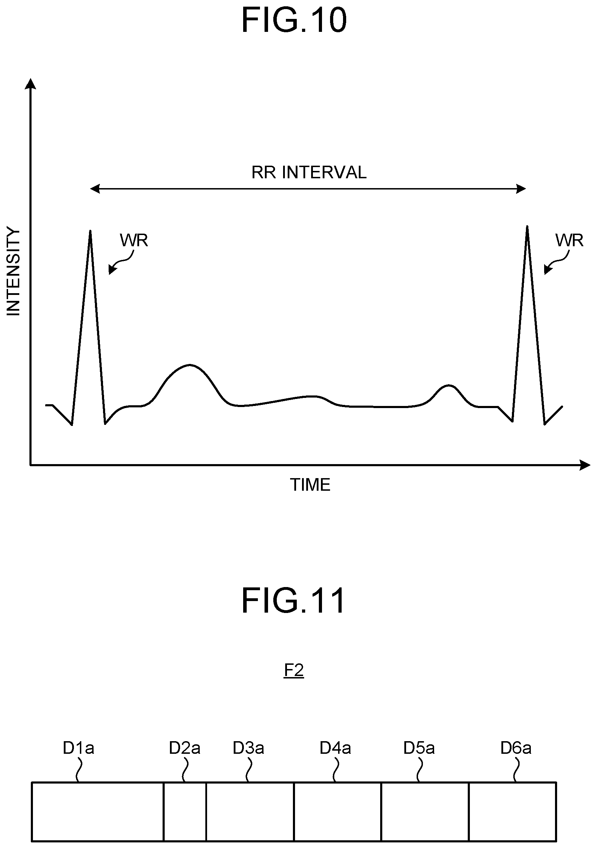

A pulse wave will be described below with reference to FIG. 10. FIG. 10 is a graph illustrating an example of a pulse wave. As illustrated in FIG. 10, the pulse wave has a waveform in which peaks called R-waves WR appear at predetermined time intervals. A pulse beat occurs due to automatic firing of pacemaker cells in a sinus node of a heart. Rhythm of the pulse beat is largely affected by both of a sympathetic nerve and a parasympathetic nerve. The sympathetic nerve acts to accelerate cardiac activity. The parasympathetic nerve acts to suppress cardiac activity. In general, the sympathetic nerve and the parasympathetic nerve act in an antagonistic manner. In a resting state or a state close to the resting state, the parasympathetic nerve acts in a dominant manner. In general, a pulse rate increases with adrenaline secretion due to excitation of the sympathetic nerve, and decreases with acetylcholine secretion due to excitation of the parasympathetic nerve. Therefore, to examine functions of the autonomic nerve, it is effective to examine variations in R-R intervals in electrocardiographs. This is described in "Normal Reference Values and Prediction Equations of Autonomic Nerve Functions Based on Variations in the R-R interval in Electrocardiographs", Fujimoto, et al. (J. Japan Diab. Sec. 30(2), 167-173, 1987) and "Heart Rate Variability and Autonomic Nerve Functions", Hayano, et al. (SEIBUTSU BUTSURI, 28-4, P32-36, 1988). The R-R interval is, as illustrated in FIG. 10, an interval between the temporally successive R waves WR. The heart rate variability is measured such that an R wave as a vertex of a QPS wave of a signal waveform is a single pulse beat. A variation of the interval between R waves in the electrocardiograph, that is, fluctuation of a duration of the R-R interval that represents the interval between the R waves in FIG. 10, is used as an autonomic nerve index. Validity of use of the fluctuation of the duration of the R-R interval as the autonomic nerve index is reported by a large number of medical institutions. Fluctuation of the duration of the R-R interval is increased in a resting state and decreased in a stress state.

Fluctuation of the duration of the R-R interval includes some kinds of characteristic fluctuation. One kind of fluctuation is fluctuation of a low-frequency component that appears at around 0.1 Hz, which is caused by variation of activity of a sympathetic nervous system due to feedback regulation of blood pressure of blood vessel. Another kind of fluctuation is fluctuation of a high-frequency component that reflects variation that is synchronized with respiration, that is, respiratory sinus arrhythmia. The high-frequency component reflects direct interference of a respiratory center to an anterior vagus nerve, stretch receptors in lungs, and baroreceptor reflex due to a change in blood pressure caused by respiration, and is mainly used as a parasympathetic nerve index that affects the heart. In other words, among waveform components that are obtained by measuring fluctuation of an R-R wave interval of the pulse wave, a power spectrum of the low-frequency component represents a degree of activity of the sympathetic nerve and a power spectrum of the high-frequency component represents a degree of activity of the parasympathetic nerve.

The fluctuation of the input pulse wave is obtained from a differential value of R-R interval values. In this case, if the differential value of the R-R interval values of the pulse wave is not data of a temporally equal interval, the activity level score calculation unit 54 converts the data to chronological data of an equal interval by using cubic spline interpolation or the like. The activity level score calculation unit 54 performs orthogonal transformation on the differential value of the R-R interval values by the fast Fourier transform or the like. Accordingly, the activity level score calculation unit 54 calculates the power spectrums of the high-frequency components and the low-frequency components of the differential value of the R-R interval values of the pulse wave. The activity level score calculation unit 54 calculates a total sum of the power spectrums of the high-frequency components as RRHF. The activity level score calculation unit 54 calculates a total sum of the power spectrums of the low-frequency components as RRLF. The activity level score calculation unit 54 calculates an autonomic nerve activity level by using Expression (1) below. The activity level score calculation unit 54 may be referred to as an autonomic nerve activity level calculation unit.

$$AN = \frac{C1 + RRLF}{C1 + RRHF} + C2 \tag{1}$$

In Expression (1), AN represents the autonomic nerve activity level, RRHF represents the total sum of the power spectrums of the high-frequency components, and RRLF represents the total sum of the power spectrums of the low-frequency components. C1 and C2 are fixed values that are defined to prevent divergence of solution.

The activity level score calculation unit 54 calculates the activity level score by using Expression (2) below.

$$NS = f(AP, R, AN) \tag{2}$$

In Expression (2), NS represents the activity level score, AP represents the behavior pattern, R represents the behavior score, and AN represents the autonomic nerve activity level. In other words, the activity level score calculation unit 54 may calculate the activity level score by using a function that includes, as parameters, the behavior pattern, the behavior score, and the autonomic nerve activity level.

Furthermore, the activity level score calculation unit 54 may calculate the activity level score by using, for example, a learning model. The learning model is an AI model that is constructed by adopting the behavior pattern, the behavior score, the autonomic nerve activity level, and the activity level score as a single data set, and performing learning by using a plurality of data sets as teacher data. In this case, the activity level score calculation unit 54 inputs the behavior pattern, the behavior score, and the autonomic nerve activity level to the learned learning model, acquires information indicating, and calculates the activity level score.

The control unit 30a provides the activity level score to the user U (Step S49). Specifically, the output control unit 50 controls at least one of the display unit 24A and the sound output unit 24B, and provides the activity level score to the user U.

The control unit 30a stores the behavior pattern and the activity level score in the storage unit 28 (Step S50). Specifically, the storage control unit 48 stores the behavior pattern identified at Step S46 and the activity level score in a predetermined format.

FIG. 11 is a diagram for explaining a format for storing the behavior pattern. As illustrated in FIG. 11, the storage format F2 may include a region D1a, a region D2a, a region D3a, a region D4a, a region D5a, and a region D6a.

In the region D1a, a numbering value of the identified behavior pattern is stored. The region D1a is configured with, for example, 3 bytes. In the region D2a, the number of dimensions of the space in which the behavior pattern information is plotted as a group is stored. The region D2a is configured with, for example, 1 byte. In the region D3a, the behavior score R of the behavior pattern information group that is determined as the behavior pattern of the user U is stored. In the region D4a, the autonomic nerve activity level is stored. The region D4a is configured with, for example, 2 bytes. In the region D5a, the activity level score is stored. The region D5a is configured with, for example, 2 bytes. The region D6a is a reserved region.

Processes at Step S51 and Step S52 are the same as the processes at Step S18 and Step S19 illustrated in FIG. 3, and therefore, explanation thereof will be omitted.

As described above, the information processing apparatus 10a according to the third embodiment is able to calculate the activity level score that indicates a degree at which the user enjoys the behavior when the user U is performing the behavior that is identified as the behavior pattern of the user U. With this configuration, the information processing apparatus 10a according to the third embodiment is able to more appropriately identify the behavior pattern of the user U.

Fourth Embodiment

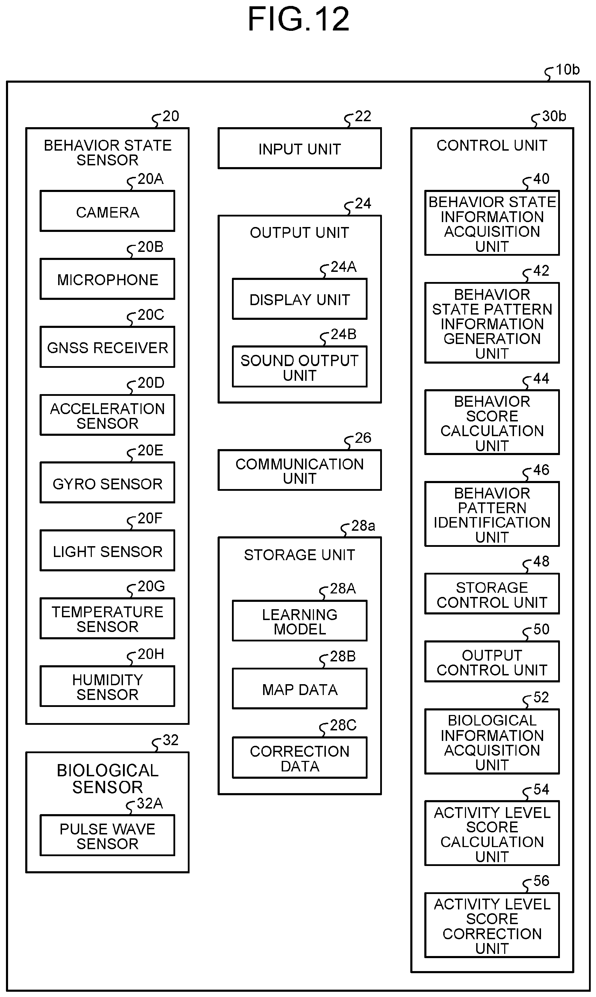

An information processing apparatus according to a fourth embodiment will be described below with reference to FIG. 12. FIG. 12 is a block diagram illustrating a configuration example of the information processing apparatus according to the fourth embodiment.

As illustrated in FIG. 12, an information processing apparatus 10b is different from the information processing apparatus 10a illustrated in FIG. 8 in that a storage unit 28a stores therein correction data 28C and a control unit 30b includes an activity level score correction unit 56.

In a different country, a region, or the like, even the same behavior pattern may be perceived in a different manner due to a difference in sensibility. The information processing apparatus 10b corrects the activity level score depending on a country or a region.

The correction data 28C is data that is used when the activity level score correction unit 56 corrects the activity level score. The correction data 28C is data with which, for example, the behavior pattern and a correction coefficient that is to be multiplied by the activity level score in accordance with a country or a region are associated.

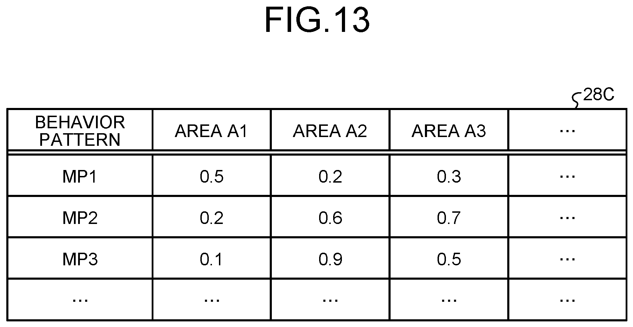

FIG. 13 is a diagram for explaining an example of the correction data. In FIG. 13, a behavior pattern MP1, a behavior pattern MP2, and a behavior pattern MP3 are illustrated as the behavior patterns. Further, an area A1, an area A2, and an area A3 are illustrated as countries or regions. In FIG. 13, the behavior patterns are described as the behavior pattern MP1 and the like as concepts, but in reality, are specifically described as "playing golf" or the like. Further, the countries or regions are described as the area A1 and the like, but in reality as concepts, are specifically described as a specific country name, such as Japan, or a specific region name, such as Tokyo.

As illustrated in FIG. 13, in the area A1, the activity level score of the behavior pattern MP1 is multiplied by 0.5, the activity level score of the behavior pattern MP2 is multiplied by 0.2, and the activity level score of the behavior pattern MP3 is multiplied by 0.1. In the area A2, the activity level score of the behavior pattern MP1 is multiplied by 0.2, the activity level score of the behavior pattern MP2 is multiplied by 0.6, and the activity level score of the behavior pattern MP3 is multiplied by 0.9. In the area A3, the activity level score of the behavior pattern MP1 is multiplied by 0.3, the activity level score of the behavior pattern MP2 is multiplied by 0.7, and the activity level score of the behavior pattern MP3 is multiplied by 0.5. As illustrated in FIG. 13, in different countries or regions, the correction coefficients to be multiplied to the activity level scores may be different even for the same behavior pattern. The correction coefficient is generated by, for example, performing questionnaire survey on behavior patterns that may be expected in each of the countries and the regions in advance. Meanwhile, in FIG. 13, the correction coefficients are classified by the regions, such as the area A1 to the area A3, but the correction coefficients may be classified by a predetermined condition instead of the regions. For example, the correction coefficients may be classified by age or gender, depending on a type of a target behavior pattern.

The activity level score correction unit 56 corrects the activity level score that is calculated by the activity level score calculation unit 54. Specifically, the activity level score correction unit 56 corrects the activity level score by using the correction data 28C, on the basis of a country or a region in which the behavior pattern is identified.

Details of process

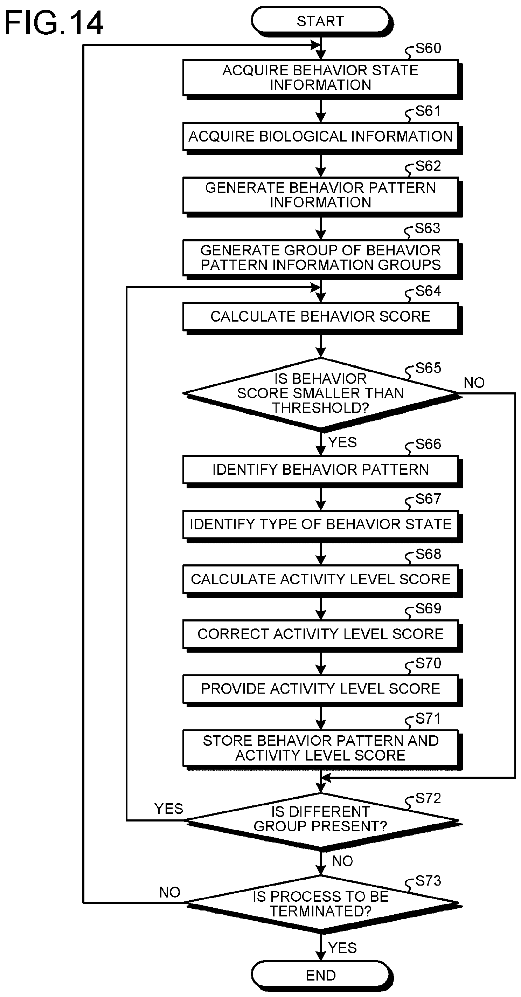

Details of a process performed by the information processing apparatus 10b according to the fourth embodiment will be described below with reference to FIG. 14. FIG. 14 is a flowchart illustrating an example of the flow of a process performed by the information processing apparatus 10b according to the fourth embodiment.

Processes at Step S60 to Step S68 are the same as the processes at Step S40 to Step S48 illustrated in FIG. 9, and therefore, explanation thereof will be omitted.

The control unit 30b corrects the activity level score (Step S69). Specifically, the activity level score correction unit 56 corrects the activity level score that is calculated by the activity level score calculation unit 54 by using the correction data 28C on the basis of the positional information that is identified by the behavior pattern identification unit 46.

The control unit 30b provides the corrected activity level score to the user U (Step S70). Specifically, the output control unit 50 controls at least one of the display unit 24A and the sound output unit 24B and provides the corrected activity level score to the user U.

The control unit 30b stores the behavior pattern and the corrected activity level score in the storage unit 28 (Step S71). Specifically, the storage control unit 48 records the behavior pattern identified at Step S66 and the corrected activity level score in a predetermined format.

Processes at Step S72 and Step S73 are the same as the processes at Step S51 and Step S52 illustrated in FIG. 9, and therefore, explanation thereof will be omitted.

As described above, the information processing apparatus 10b according to the fourth embodiment multiplies the activity level score by the correction coefficient in accordance with a country or a region, and corrects the activity level score. With this configuration, the information processing apparatus 10b according to the fourth embodiment is able to more appropriately correct the activity level score in accordance with a country or a region.

Modification of Fourth Embodiment

A modification of the fourth embodiment will be described below. In the fourth embodiment, as illustrated in FIG. 13, the activity level score is calculated by using the correction data 28C in which the behavior pattern and the area are associated with each other. However, in the correction data, the behavior pattern need not always be associated. Specifically, it may be possible to determine a special correction coefficient for each area. In this case, the activity level score correction unit 56 may correct the autonomic nerve activity level calculated by the activity level score calculation unit 54, on the basis of the correction data. In this case, the activity level score correction unit 56 may be referred to as an autonomic nerve activity level correction unit.

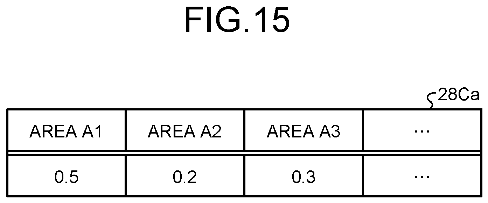

FIG. 15 is a diagram for explaining an example of the correction data. As illustrated in FIG. 15, in correction data 28Ca, the correction coefficient is determined for each area. In the example illustrated in FIG. 15, the correction coefficient of the area A1 is 0.5, the correction coefficient of the area A2 is 0.2, and the correction coefficient of the area A3 is 0.3.

For example, if it is determined that the region in which the autonomic nerve activity level of the user U is calculated is the area A1, the activity level score correction unit 56 corrects the autonomic nerve activity level by multiplying the calculated autonomic nerve activity level by 0.5. With this configuration, in the modification of the fourth embodiment, it is possible to more appropriately calculate the autonomic nerve activity level depending on each region.

Fifth Embodiment

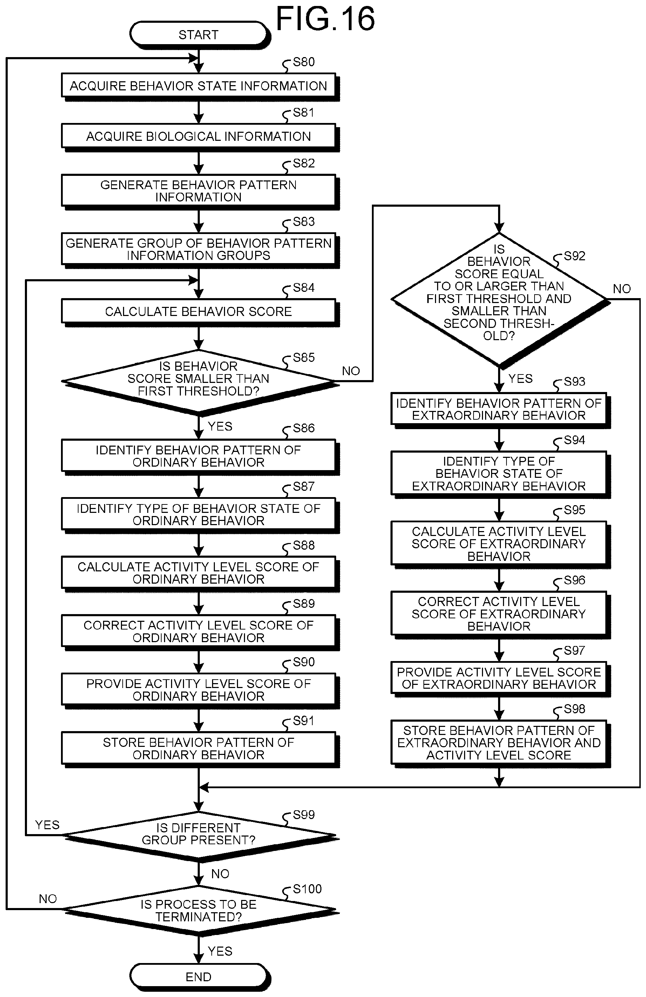

A fifth embodiment will be described below. FIG. 16 is a flowchart illustrating an example of the flow of a process performed by the information processing apparatus according to the fifth embodiment. A configuration of the information processing apparatus according to the fifth embodiment is the same as the configuration of the information processing apparatus 10b illustrated in FIG. 12, and therefore, explanation thereof will be omitted.

In the fifth embodiment, the information processing apparatus 10b separately calculates the activity level score for the identified behavior pattern of the ordinary behavior and the identified behavior pattern of the extraordinary behavior of the user U. Further, in the fifth embodiment, the information processing apparatus 10b separately corrects the calculated activity level score of the behavior pattern of the ordinary behavior and the calculated activity level score of the behavior pattern of the extraordinary behavior of the user U.

Processes at Step S80 to Step S84 are the same as the processes at Step S40 to Step S44 illustrated in FIG. 9, and therefore, explanation thereof will be omitted.

Processes at Step S85 to Step S87 are the same as the processes at Step S24 to Step S26 illustrated in FIG. 6, and therefore, explanation thereof will be omitted.

The control unit 30b calculates the activity level score of the behavior pattern of the ordinary behavior (Step S88). Specifically, the activity level score calculation unit 54 calculates the activity level score of the behavior pattern of the ordinary behavior of the user U on the basis of the pulse wave information acquired at Step S81.

The control unit 30b corrects the activity level score of the behavior pattern of the ordinary behavior (Step S89). Specifically, the activity level score correction unit 56 corrects the activity level score of the behavior pattern of the ordinary behavior that is calculated by the activity level score calculation unit 54, by using the correction data 28C on the basis of the positional information that is identified by the behavior pattern identification unit 46.

The control unit 30b provides the corrected activity level score of the behavior pattern of the ordinary behavior to the user U (Step S90). Specifically, the output control unit 50 controls at least one of the display unit 24A and the sound output unit 24B, and provides the corrected activity level score to the user U.

The control unit 30b stores the corrected activity level score of the behavior pattern of the ordinary behavior in the storage unit 28 (Step S91). Specifically, the storage control unit 48 records the behavior pattern of the ordinary behavior that is identified at Step S86 and the corrected activity level score in a predetermined format.

If it is determined as No at Step S85, the process goes to Step S92. Processes at Step S92 to Step S94 are the same as the processes at Step S28 to Step S30 illustrated in FIG. 6, and therefore, explanation thereof will be omitted.

The control unit 30b calculates the activity level score of the behavior pattern of the extraordinary behavior (Step S95). Specifically, the activity level score calculation unit 54 calculates the activity level score of the behavior pattern of the extraordinary behavior of the user U on the basis of the pulse wave information that is acquired at Step S81.

The control unit 30b corrects the activity level score of the behavior pattern of the extraordinary behavior (Step S96). Specifically, the activity level score correction unit 56 corrects the activity level score of the behavior pattern of the extraordinary behavior that is calculated by the activity level score calculation unit 54, by using the correction data 28C on the basis of the positional information that is identified by the behavior pattern identification unit 46.

The control unit 30b provides the corrected activity level score of the behavior pattern of the extraordinary behavior to the user U (Step S97). Specifically, the output control unit 50 controls at least one of the display unit 24A and the sound output unit 24B, and provides the corrected activity level score to the user U.

The control unit 30b stores the behavior pattern of the extraordinary behavior and the corrected activity level score in the storage unit 28 (Step S91). Specifically, the storage control unit 48 stores the behavior pattern of the extraordinary behavior that is identified at Step S86 and the corrected activity level score in a predetermined format.

Processes at Step S99 and Step S100 are the same as the processes at Step S51 and Step S52 illustrated in FIG. 9, and therefore, explanation thereof will be omitted.

As described above, the information processing apparatus 10b according to the fifth embodiment is able to separately calculate the activity level score in each of a case where a behavior that is identified as the behavior pattern of the ordinary behavior is performed and a case where a behavior that is identified as the behavior pattern of the extraordinary behavior is performed. With this configuration, the information processing apparatus 10b according to the fifth embodiment is more appropriately identify the behavior pattern of the user U.

Furthermore, the information processing apparatus 10b according to the fifth embodiment multiplies the activity level score by the correction coefficient in accordance with a country or a region and corrects the activity level score of the behavior pattern of the ordinary behavior and the activity level score of the behavior pattern of the extraordinary behavior. With this configuration, the information processing apparatus 10b according to the fifth embodiment is able to more appropriately correct the activity level score in accordance with a country or a region.

Sixth Embodiment

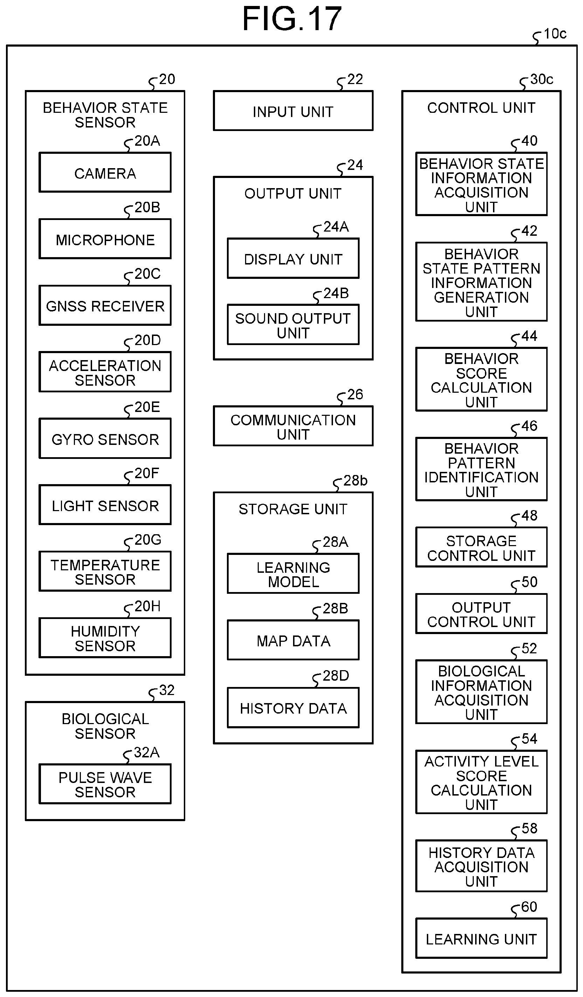

An information processing apparatus according to a sixth embodiment will be described below with reference to FIG. 17. FIG. 17 is a block diagram illustrating a configuration example of the information processing apparatus according to the sixth embodiment.

As illustrated in FIG. 17, an information processing apparatus 10c is different from the information processing apparatus 10a illustrated in FIG. 8 in that a storage unit 28b includes history data 28D and a control unit 30c includes a history data acquisition unit 58 and a learning unit 60.

Users may have different hobbies and interests, so that even when the users are performing the same behaviors, a value of the activity level score may vary for each of the users. The information processing apparatus 10c according to the sixth embodiment calculates the activity level score by using a learned model that is customized for each of the users.

The history data 28D is data related to a history of activity level scores. The history data 28D may include information on ranks of the activity level scores in a predetermined period for each user. Specifically, the history data 28D may include information on the behavior pattern for which the activity level score is higher than a predetermined rank in the predetermined period. The predetermined period is, for example, three months, but not limited thereto.

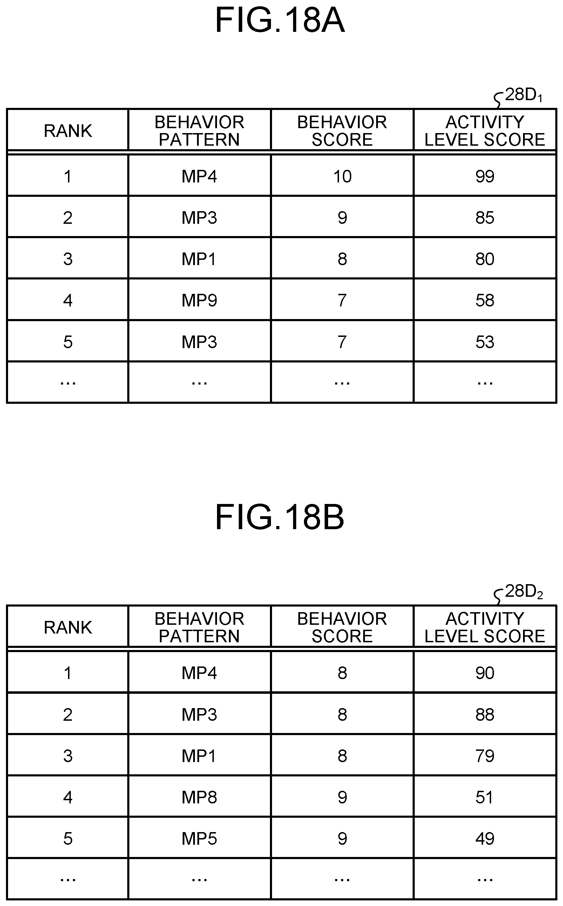

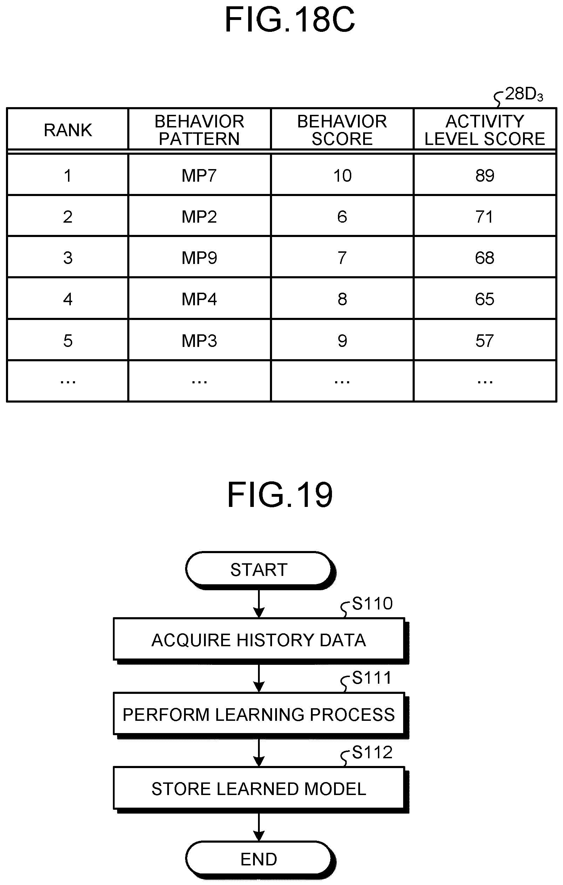

FIG. 18A to FIG. 18C are diagrams for explaining examples of the history data 28D. As illustrated in FIG. 18A to FIG. 18C, in the history data 28D, the rank, the behavior pattern, the behavior score, and the activity level score are associated with one another. FIG. 18A illustrates an example of history data of a user U1. FIG. 18B illustrates an example of history data of a user U2. FIG. 18C illustrates an example of history data of a user U3. FIG. 18A to FIG. 18C illustrate behavior patterns at a first rank to a fifth rank for which the activity level scores are high in the predetermined period for the respective users U1 to U3.

As illustrated in FIG. 18A, the first rank of the user U1 is a behavior pattern MP4 with the behavior score of 10 and the activity level score of 99. The second rank of the user U1 is a behavior pattern MP3 with the behavior score of 9 and the activity level score of 85. The third rank of the user U1 is a behavior pattern MP1 with the behavior score of 8 and the activity level score of 80. The fourth rank of the user U1 is a behavior pattern MP9 with the behavior score of 7 and the activity level score of 58. The fifth score of the user U1 is a behavior pattern MP3 with the behavior score of 7 and the activity level score of 53.

As illustrated in FIG. 18B, the first rank of the user U2 is the behavior pattern MP4 with the behavior score of 8 and the activity level score of 90. The second rank of the user U2 is the behavior pattern MP3 with the behavior score of 8 and the activity level score of 88. The third rank of the user U2 is the behavior pattern MP1 with the behavior score of 8 and the activity level score of 79. The fourth rank of the user U2 is a behavior pattern MP8 with the behavior score of 9 and the activity level score of 51. The fifth rank of the user U2 is a behavior pattern MP5 with the behavior score of 9 and the activity level score of 49.

As illustrated in FIG. 18C, the first rank of the user U3 is a behavior pattern MP7 with the behavior score of 10 and the activity level score of 89. The second rank of the user U3 is a behavior pattern MP2 with the behavior score of 6 and the activity level score of 71. The third score of the user U3 is the behavior pattern MP9 with the behavior score of 7 and the activity level score of 68. The fourth rank of the user U3 is the behavior pattern MP4 with the behavior score of 8 and the activity level score of 65. The fifth rank of the user U3 is the behavior pattern MP3 with the behavior score of 9 and the activity level score of 57.

As illustrated in FIG. 18A to FIG. 18C, even for the same behavior pattern, the behavior pattern with the high activity level score varies for each of the users, and the activity level score varies between individuals.

The history data acquisition unit 58 acquires the history data 28D from a storage unit 28c. Specifically, the history data acquisition unit 58 acquires the history data 28D of a certain user for whom the activity level score is to be calculated.

The learning unit 60 generates a learned model for calculating the activity level score of the user through learning by machine learning based on learning data. In the present embodiment, the learning unit 60 generates a learned model for calculating the activity level score on the basis of the history data 28D that is acquired by the history data acquisition unit 58, for example. The learning unit 60 performs learning on a weight of a deep neural network (DNN) as the learned model for calculating the activity level score, for example. The learning unit 60 may perform learning by using a well-known machine learning method, such as deep learning, for example. The learning unit 60 may update the learned model every time the learning data is updated, for example.

A learning process according to the sixth embodiment will be described below with reference to FIG. 19. FIG. 19 is a flowchart illustrating an example of the flow of the learning process according to the sixth embodiment.

The control unit 30c acquires the learning data (Step S110). Specifically, the history data acquisition unit 58 acquires, from the storage unit 28b, the history data 28D corresponding to the predetermined period for the user for whom the activity level score is to be calculated. The history data 28D acquired by the history data acquisition unit 58 may include information on at least the rank, the behavior pattern, the behavior score, and the activity level score. The history data acquisition unit 58 acquires the history data 28D including the first rank to the 1000-th rank in the past three months, for example.

The control unit 30c performs the learning process (Step S111). Specifically, the learning unit 60 generates, through machine learning, the learned model for calculating the activity level score of the user, by using the history data 28D that is acquired by the history data acquisition unit 58. More specifically, the learning unit 60 generates the learned model through learning by adopting the behavior pattern, the behavior score, the autonomic nerve activity level, and the activity level score as a single data set, and performing learning using a plurality of (for example, 1000) data sets as teacher data. The learning unit 60 generates the learned model for each user for whom the activity level score is to be calculated, for example. In other words, in the present embodiment, the learned model that is customized for each of the users is generated.

The control unit 30c stores the learned model (Step S112). Specifically, the learning unit 60 stores the generated learned model in the storage unit 28c.

Details of process

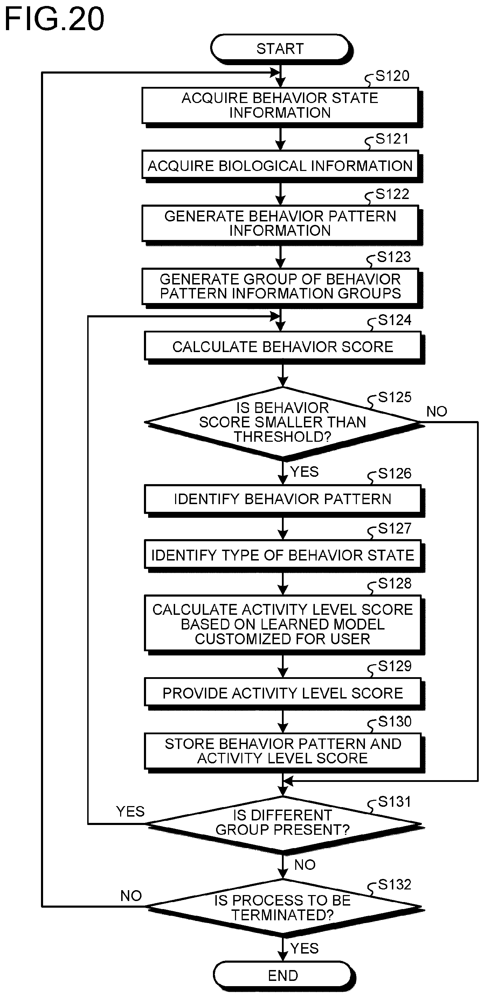

Details of a process performed by the information processing apparatus 10c according to the sixth embodiment will be described below with reference to FIG. 20. FIG. 20 is a flowchart illustrating an example of the flow of a process performed by the information processing apparatus 10c according to the sixth embodiment.

Processes from Step S120 to Step S127 are the same as the processes from Step S40 to Step S47 illustrated in FIG. 9, and therefore, explanation thereof will be omitted.

The control unit 30c calculates the activity level score on the basis of the learned model corresponding to the user (Step S128). Specifically, the activity level score calculation unit 54 calculates the activity level score of the user by using the learned model that is customized for the user.

Processes from Step S129 to Step S132 are the same as the processes from Step S49 to Step S52 illustrated in FIG. 9, and therefore, explanation thereof will be omitted.

As described above, the information processing apparatus 10c according to the sixth embodiment calculates the activity level scores of the user U1 to the user U3 by using the learned model that is generated and customized in accordance with a history of the activity level scores for each of the users, such as the user U1 to the user U3. With this configuration, the information processing apparatus 10c according to the sixth embodiment is able to more appropriately calculate the activity level score in accordance with the sensibility of the user.

Seventh Embodiment

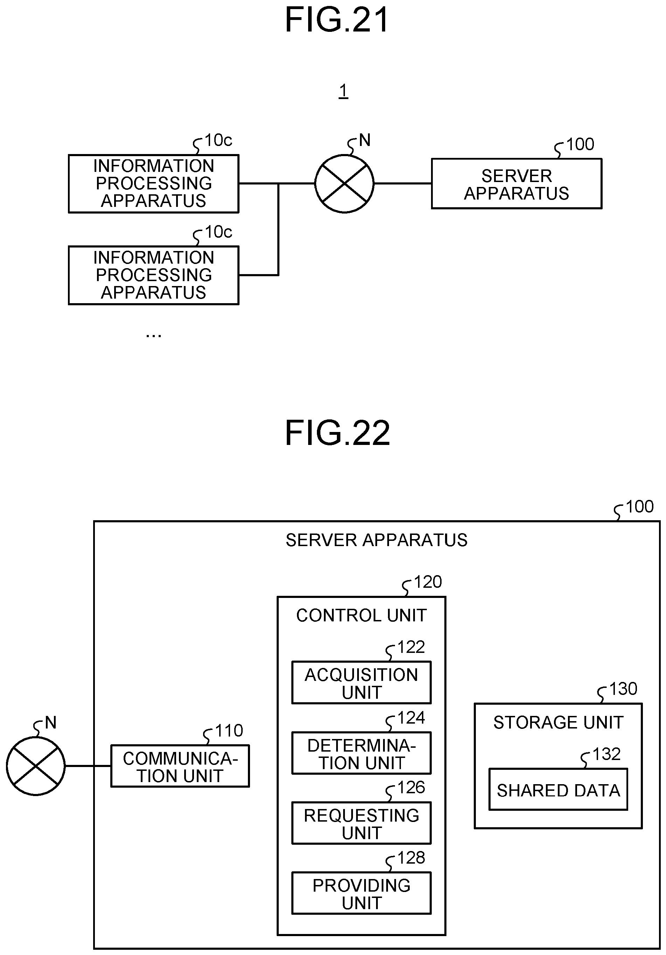

An information processing system according to a seventh embodiment will be described below with reference to FIG. 21. FIG. 21 is a diagram for explaining a configuration example of the information processing system according to the seventh embodiment.

As illustrated in FIG. 21, an information processing system 1 according to the seventh embodiment includes the plurality of information processing apparatuses 10c, and a server apparatus 100. The information processing apparatuses 10c and the server apparatus 100 are communicably connected to one another via a network N (for example, the Internet). In other words, the information processing system 1 according to the seventh embodiment is configured such that the information processing apparatuses 10c according to the sixth embodiment and the server apparatus 100 are communicably connected to one another.

In the sixth embodiment as described above, the activity level of the user is calculated by using the learned model that is generated and customized in accordance with the history of the activity level scores for each user. In the seventh embodiment, the server apparatus 100 stores pieces of history data of a plurality of users as shared data, generates a learned model on the basis of pieces of history data of a plurality of users who have similar activity level scores and tendencies of behavior patterns, and calculates the activity level of a user.

A configuration of the server apparatus according to the seventh embodiment will be described with reference to FIG. 22. FIG. 22 is a block diagram illustrating a configuration example of the server apparatus according to the seventh embodiment.

As illustrated in FIG. 22, the server apparatus 100 includes a communication unit 110, a control unit 120, and a storage unit 130. The server apparatus 100 is what is called a cloud server.

The communication unit 110 is implemented by, for example, a network interface card (NIC), a communication circuit, or the like. The communication unit 110 is connected to the network N in a wired or wireless manner, and transmits and receives information to and from the information processing apparatuses 10c.

The control unit 120 controls operation of each of the units of the server apparatus 100. The control unit 120 is implemented by, for example, causing a CPU, an MPU, or the like to execute a program that is stored in a storage unit (not illustrated) by using a RAM or the like as a work area. The control unit 120 may be implemented by, for example, an integrated circuit, such as an ASIC or an FPGA. The control unit 120 may be implemented by a combination of a hardware and software. The control unit 120 includes an acquisition unit 122, a determination unit 124, a requesting unit 126, and a providing unit 128.

The acquisition unit 122 acquires, from the communication unit 110, the history data related to a history of the activity level scores of each of the users who are wearing the information processing apparatuses 10c, for example. The acquisition unit 122 acquires, for example, history data $28D_3$ to history data $28D_1$ of the user U1 to the user U3 illustrated in FIG. 18A. The acquisition unit 122 stores the acquired history data as shared data 132 in the storage unit 130.

The determination unit 124 determines a tendency of the history data in the shared data 132. The determination unit 124 determines whether users with similar tendencies of the history data are present, for example.

If a plurality of users with similar tendencies of history data with are present, the requesting unit 126 asks whether it is possible to allow a different user to use the subject history data.

If use of the history data is approved, the providing unit 128 provides the history data to the user with the similar tendency of the history data.

The storage unit 130 is a memory for storing various kinds of information, such as contents of calculation performed by the control unit 120 and a program, and includes at least one of a main storage device, such as a RAM or a ROM, and an external storage device, such as an HDD, for example.

The storage unit 130 stores therein the shared data 132. The shared data 132 may include pieces of the history data related to the activity level scores of the plurality of users who are wearing the information processing apparatus 10c. The shared data 132 may include the history data $28D_1$ to the history data $28D_3$ of the user U1 to the user U3 illustrated in FIG. 18A to FIG. 18C, for example.

Process performed by server apparatus

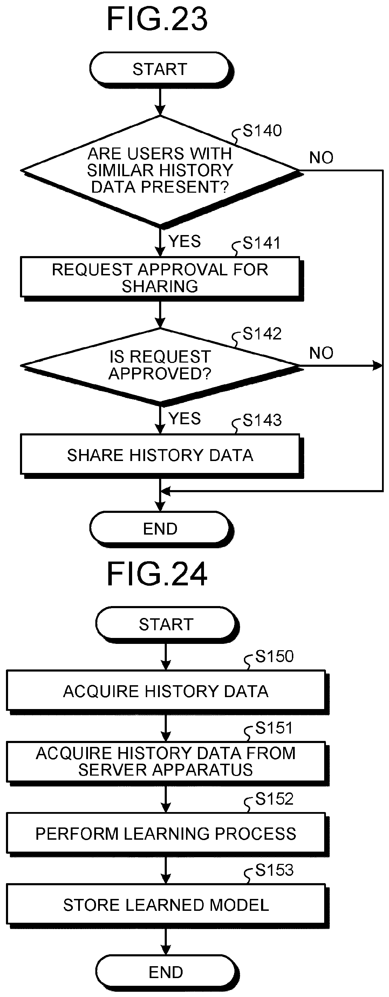

A process performed by the server apparatus according to the seventh embodiment will be described below with reference to FIG. 23. FIG. 23 is a flowchart illustrating an example of the flow of a process performed by the server apparatus according to the seventh embodiment.

The control unit 120 refers to the shared data 132 and determines whether users with similar history data are present (Step S140). For example, it is assumed that the shared data 132 includes the history data $28D_1$ to the history data $28D_3$ of the user U1 to the user U3 illustrated in FIG. 18A to FIG. 18C. For example, the determination unit 124 determines, as users with similar history data, users for whom the behavior patterns at the first rank to the third rank are the same, for whom the behavior scores of each of the behavior patterns are equal to or larger than eight, and for whom differences between the activity level scores are equal to or smaller than 10. In this case, the determination unit 124 identifies that the first rank of the activity level score of each of the user U1 and the user U2 is the behavior pattern MP4, the second rank is the behavior pattern MP3, and the third rank is the behavior pattern MP1. The determination unit 124 identifies that, as for the user U1, the behavior score of the behavior pattern MP4 is 10, the behavior score of the behavior pattern MP3 is 9, and the behavior score of the behavior pattern MP1 is 8. The determination unit 124 identifies that, as for the user U2, the behavior score of the behavior pattern MP4 is 8, the behavior score of the behavior pattern MP3 is 8, and the behavior score of the behavior pattern MP1 is 8. The determination unit 124 identifies that, as for the user U1 and the user U2, a difference in the activity level score of the behavior pattern MP1 is 9, a difference in the activity level score of the behavior pattern MP3 is 3, and a difference in the activity level score of the behavior pattern MP1 is 1. In this case, the determination unit 124 determines that the user U1 and the user U2 have similar history data.

In the present embodiment, the case has been described in which the determination unit 124 selects two from the three users, that is the user U1 to the user U3, but this is described by way of example, and the number of users in an actual population is not specifically limited. The determination unit 124 may determine that three or more users have similar history data. The determination unit 124 may determine whether pieces of the history data are similar to each other by a method other than the method that is described in the present embodiment. The determination unit 124 may determine whether the pieces of history data are similar to each other in accordance with a predetermined conditional expression that is mathematically defined, for example.

If it is determined that the similar users are present (Step S140; Yes), the process goes to Step S141. If it is determined that the similar users are not present (Step S140; No), the process in FIG. 23 is terminated.

If it is determined as Yes at Step S140, the control unit 120 requests the users with the similar the history data to approve sharing (Step S141). Specifically, the requesting unit 126 transmits a notice for requesting approval for sharing of the history data to the user U1 and the user U2 via the communication unit 110.

The control unit 120 determines whether the request for sharing is approved (Step S142). Specifically, the requesting unit 126 determines whether a replay indicating approval of sharing of the history data is provided from the user U1 or the user U2 in response to the request for sharing the history data, which is transmitted at Step S141. If it is determined that the request for sharing is approved (Step S142; Yes), the process goes to Step S143. If it is determined that the request for sharing is not approved (Step S142; No), the process in FIG. 23 is terminated.

If it is determined as Yes at Step S142, the control unit 120 shares the history data (Step S143). Specifically, for example, if the user U2 has approved sharing of the history data, the providing unit 128 transmits the history data $28D_1$ of the user U1 to the information processing apparatus 10c that is worn on the user U1 via the communication unit 110. Then, the process in FIG. 23 is terminated.

The learning process according to the seventh embodiment will be described below with reference to FIG. 24. FIG. 24 is a flowchart illustrating an example of the flow of the learning process according to the seventh embodiment. In the following, the process in which the information processing apparatus 10c worn on the user U1 acquires history data $28D_2$ of the user U2 and performs the learning process will be described.

The control unit 30c acquires the history data (Step S150). Specifically, the history data acquisition unit 58 acquires, from the storage unit 28c, the history data $28D_1$ corresponding to the predetermined period for the user for whom the activity level score is to be calculated. The history data $28D_1$ that is acquired by the history data acquisition unit 58 may include information on at least the rank, the behavior pattern, the behavior score, and the activity level score. The history data acquisition unit 58 acquires the history data $28D_1$ including the first rank to the 1000-th rank in past three months, for example. Specifically, the history data acquisition unit 58 acquires 1000 data sets as teacher data.

The control unit 30c acquires, from the server apparatus 100, the history data that is similar to the history data of the user U1 (Step S151). Specifically, the history data acquisition unit 58 acquires the history data $28D_2$ of the user U2 from the server apparatus 100 via the communication unit 26, for example. Here, for example, it may be possible that a small number of behavior patterns are included in the 1000 data sets of the history data $28D_1$. For example, when the learned model is to be generated, in some cases, 6000 to 8000 data sets or more may be needed. In the present embodiment, the history data acquisition unit 58 acquires the history data $28D_2$ that is similar to the history data $28D_1$ from the server apparatus 100, so that it is possible to compensate for the number of the data sets and generate a more appropriate learned model.

The control unit 30c performs the learning process (Step S152). Specifically, the learning unit 60 performs learning through machine learning, and generates the learned model for calculating the activity level score of the user by using the history data $28D_1$ and the history data $28D_2$ that are acquired by the history data acquisition unit 58.

The control unit 30c stores the learned model (Step S153). Specifically, the learning unit 60 stores the generated learned model in the storage unit 28b. Then, the process in FIG. 24 is terminated.

Details of process

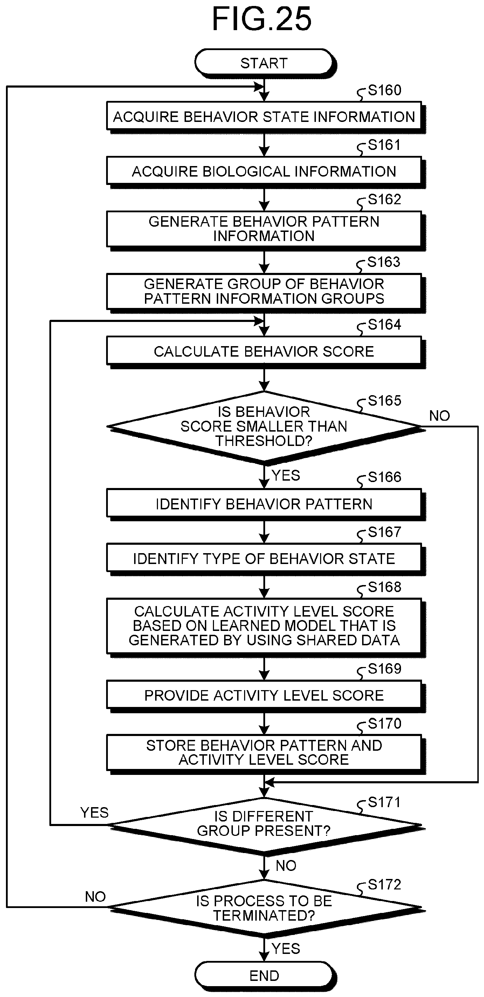

Details of a process performed by the information processing apparatus 10c will be described below with reference to FIG. 25. FIG. 25 is a flowchart illustrating an example of the flow of a process performed by the information processing apparatus 10c according to the seventh embodiment.

Processes from Step S160 to Step S167 are the same as the processes from Step S120 to Step S127 illustrated in FIG. 20, and therefore, explanation thereof will be omitted.

The control unit 30c calculates the activity level score on the basis of the learned model that is generated by using the shared data (Step S168). Specifically, the activity level score calculation unit 54 calculates the activity level score of the user U1 by using the learned model that is generated by using the history data $28D_1$ and the history data $28D_2$.

Processes from Step S169 to Step S172 are the same as the processes from Step S129 to Step S132 illustrated in FIG. 20, and therefore, explanation thereof will be omitted.

As described above, the information processing apparatus 10c according to the seventh embodiment generates the learned model by using the history data $28D_1$ of the user U1 and the history data $28D_2$ of the user U2 that is similar to the history data $28D_1$, and calculates the activity level score by using the learned model. With this configuration, the information processing apparatus 10c according to the seventh embodiment is more appropriately calculate the activity level score.

Eighth Embodiment

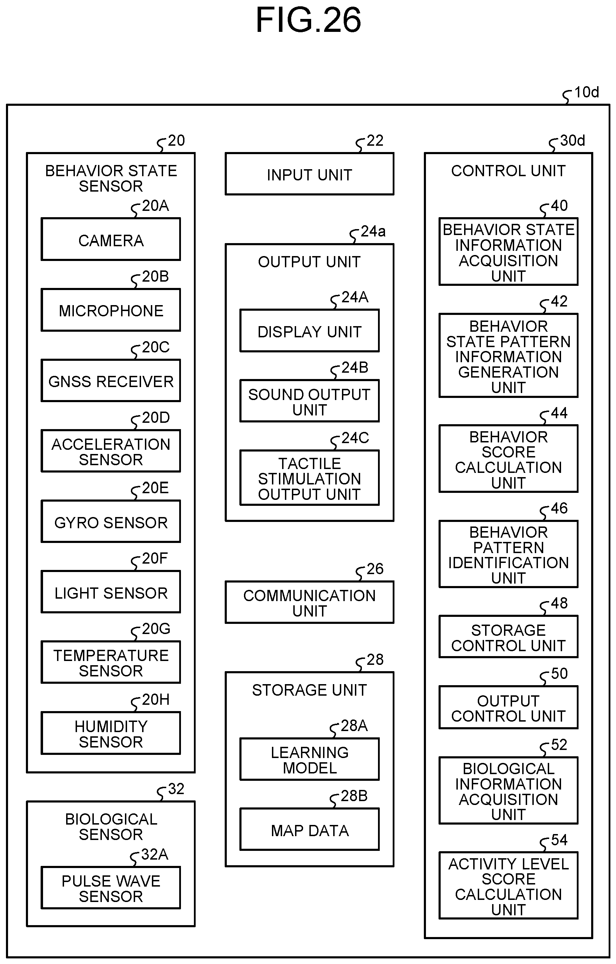

An information processing apparatus according to an eighth embodiment will be described below with reference to FIG. 26. FIG. 26 is a block diagram illustrating a configuration example of the information processing apparatus according to the eighth embodiment.

As illustrated in FIG. 26 , an information processing apparatus 10d is different from the information processing apparatus 10 illustrated in FIG. 2 in that an output unit 24a includes a tactile stimulation output unit 24C.

The biological information on the user changes from time to time, and therefore, the activity level score of the user may change depending on a change of the biological information. The information processing apparatus 10d provides a change of the activity level score in an easily understandable manner by providing a temporal change of the activity level score of the user in a comparative manner.

The tactile stimulation output unit 26C is a device that outputs tactile stimulation to the user U. For example, the tactile stimulation output unit 26C outputs the tactile stimulation to the user by physical operation, such as vibration; however, a type of the tactile stimulation is not limited to vibration, and may be arbitrary stimulation.

The output control unit 50 of a control unit 30d of the information processing apparatus 10d according to the eighth embodiment causes the output unit 24a to output information indicating a temporal change of the activity level score that is calculated by the activity level score calculation unit 54. In other words, the output control unit 50 causes the output unit 24a to outputs information indicating a relative change of the activity level score.

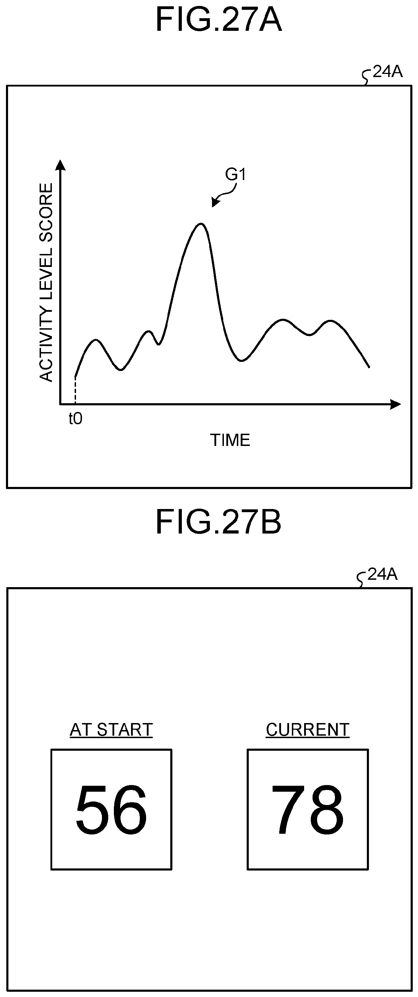

A method of displaying a temporal change of the activity level score in a comparative manner will be described below with reference to FIG. 27A to FIG. 27E. FIG. 27A to FIG. 27E are diagrams for explaining the method of displaying a temporal change of the activity level score in a comparative manner.

As illustrated in FIG. 27A, the output control unit 50 causes the display unit 24A to display a temporal change of the activity level score as a graph G1, for example. In FIG. 27A, the horizontal axis represents a time and the vertical axis represents the activity level score. In the graph G1, a time t0 indicates a time point at which calculation of the activity level score is started. A user is able to easily recognize the temporal change of the activity level score by viewing the graph G1.

As illustrated in FIG. 27B, the output control unit 50 causes the display unit 24A to display a value of the activity level score at the start and a value of a current activity level score side by side, for example. In the example illustrated in FIG. 27B, it is indicated that the activity level score at the start is 56 and the current activity level score is 78. With this configuration, the user is able to easily recognize the temporal change of the activity level score.

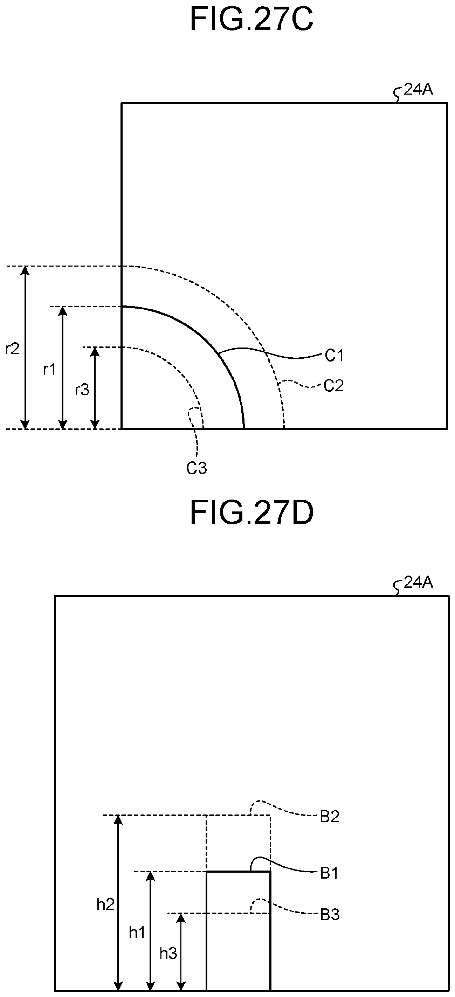

As illustrated in FIG. 27C, the output control unit 50 causes the display unit 24A to display circular arcs by equally dividing a circle centered at a lower left corner of a screen into four arcs, for example. In the example illustrated in FIG. 27C, a circular arc C1 represents the activity level score at the start, and a radius r1 represents a magnitude of the activity level score. In the example illustrated in FIG. 27C, a circular arc C2 and a circular arc C3 represent current activity level scores. If the current activity level score is increased from the start, the output control unit 50 causes the display unit 24A to display the circular arc C2 with a radius r2 that is larger than the radius r1, together with the circular arc C1 with the radius r1. If the current activity level score is reduced from the start, the output control unit 50 causes the display unit 24A to display the circular arc C3 with a radius r3 that is smaller than the radius r1, together with the circular arc C1 with the radius r1. With this configuration, the user is able to easily recognize the temporal change of the activity level score.

As illustrated in FIG. 27D, the output control unit 50 causes the display unit 24A to display a bar, for example. In the example illustrated in FIG. 27D, a bar B1 is displayed in a central portion of the display unit 24A, but embodiments are not limited to this example, and the bar B1 may be displayed at a lower left corner or a lower right corner. In the example illustrated in FIG. 27D, the bar B1 represents the activity level score at the start, and a height h1 represents the magnitude of the activity level score. In the example illustrated in FIG. 27D, a bar B2 and a bar B3 represent current activity level scores. If the current activity level score is increased from the start, the output control unit 50 causes the display unit 24A to display the bar B2 with a height h2 that is larger than the height h1, together with the bar B1 with the height h1. If the current activity level score is reduced from the start, the output control unit 50 causes the display unit 24A to display the bar B3 with a height h3 that is smaller than the height h1, together with the bar B1 with the height h1. With this configuration, the user is able to easily recognize the temporal change of the activity level score.

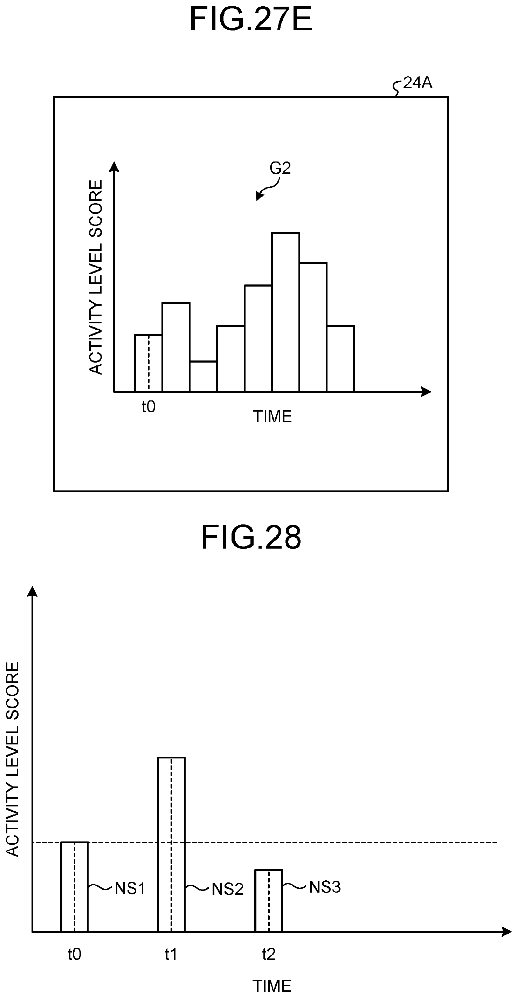

As illustrated in FIG. 27E, the output control unit 50 causes the display unit 24A to display a graph G2, for example. The graph G2 may be a bar chart. In FIG. 27E, the horizontal axis represents a time and the vertical axis represents the activity level score. In the graph G2, a time t0 indicates a time point at which calculation of the activity level score is started. The user is able to easily recognize a temporal change of the activity level score by viewing the graph G2.

The output control unit 50 may cause the sound output unit 24B or the tactile stimulation output unit 26C to output information indicating a temporal change of the activity level score, for example.

A method of outputting the information indicating the temporal change of the activity level score by the sound output unit 24B or the tactile stimulation output unit 24C will be described below with reference to FIG. 28. FIG. 28 is a diagram for explaining the method of outputting the information indicating the temporal change of the activity level score by the sound output unit 24B or the tactile stimulation output unit 24C.

In FIG. 28, the horizontal axis represents a time and the vertical axis represents the activity level score. An activity level score NS1 at a time t0 is the activity level score at the time point at which calculation of the activity level score is started. In the example illustrated in FIG. 28, the activity level score NS1 is used as a reference. The output control unit 50 sets, for example, a volume of sound for outputting the activity level score NS1 as a reference from the sound output unit 24B. The output control unit 50 sets, for example, magnitude of stimulation (for example, vibration) indicating the activity level score NS1 as a reference to be output from the tactile stimulation output unit 24C.

Here, at a time t1, it is assumed that the activity level score is changed to the activity level score NS2 that is larger than the activity level score NS1. In this case, the output control unit 50 causes the sound output unit 24B to output a set of sound corresponding to the activity level score NS1 and sound corresponding to the activity level score NS2, for example. The output control unit 50 causes the tactile stimulation output unit 24C to output a set of stimulation corresponding to the activity level score NS1 and stimulation corresponding to the activity level score NS2, for example. The sound corresponding to the activity level score NS2 is louder than the sound corresponding to the activity level score NS1. The stimulation corresponding to the activity level score NS2 is stronger than the stimulation corresponding to the activity level score NS1. It is preferable to change the volume of the sound corresponding to the activity level score NS2 in accordance with a ratio of the activity level score NS2 to the activity level score NS1, for example. It is preferable to change the magnitude of the stimulation the activity level score NS2 in accordance with a ratio of the activity level score NS2 to the activity level score NS1, for example. With this configuration, the user is able to recognize magnitude of the activity level score NS2 relative to the activity level score NS1.

Here, at a time t2, it is assumed that the activity level score is changed to an activity level score NS3 that is smaller than the activity level score NS1. In this case, the output control unit 50 causes the sound output unit 24B to output a set of sound corresponding to the activity level score NS1 and sound corresponding to the activity level score NS3, for example. The output control unit 50 causes the tactile stimulation output unit 24C to output stimulation corresponding to the activity level score NS1 and stimulation corresponding to the activity level score NS3, for example. The sound corresponding to the activity level score NS3 is lower than the sound corresponding to the activity level score NS1. The stimulation corresponding to the activity level score NS3 is weaker than the stimulation corresponding to the activity level score NS1. It is preferable to change the volume of the sound corresponding to the activity level score NS3 in accordance with a ratio of the activity level score NS2 to the activity level score NS1, for example. It is preferable to change the magnitude of the stimulation of the activity level score NS3 in accordance with a ratio of the activity level score NS2 to the activity level score NS1, for example. With this configuration, the user is able to recognize the magnitude of the activity level score NS2 relative to the activity level score NS1.

As described above, the information processing apparatus 10d according to the eighth embodiment provides a temporal change of the activity level score of the user in a comparative manner. With this configuration, the information processing apparatus 10d according to the eighth embodiment makes it possible to easily recognize a temporal change of the activity level score.

Thus, the embodiments of the present disclosure have been described above, but the present disclosure is not limited by the details of the embodiments. Further, the components described above include one that can easily be thought of by a person skilled in the art, one that is practically identical, and one that is within an equivalent range. Furthermore, the components described above may be combined appropriately. Moreover, within the scope not departing from the gist of the embodiments described above, various omission, replacement, and modifications of the components may be made.

Industrial Applicability

The information processing apparatus, the information processing method, and the program according to the present disclosure may be applied to a technique for analyzing a behavior of a user.

Reference Signs List

10, 10a, 10b, 10c, 10d Information processing apparatus
20 Behavior state sensor
20A Camera
20B Microphone
20C GNSS receiver
20D Acceleration sensor
20E Gyro sensor
20F Light sensor
20G Temperature sensor
20H Humidity sensor
22 Input unit
24 Output unit
24A Display unit
24B Sound output unit

24C Tactile stimulation output unit
26, 110 Communication unit
28, 130 Storage unit
28A Learning model
28B Map data
28C, 28Ca Correction data
28D History data
30, 30a, 30b, 30c, 30d, 120 Control unit
32 Biological sensor
32A Pulse wave sensor
40 Behavior state information acquisition unit
42 Behavior pattern information generation unit
44 Behavior score calculation unit
46 Behavior pattern identification unit
48 Storage control unit
50 Output control unit
52 Biological information acquisition unit
54 Activity level score calculation unit
56 Activity level score correction unit
58 History data acquisition unit
60 Learning unit
100 Server apparatus
122 Acquisition unit
124 Determination unit
126 Requesting unit
128 Providing unit

**Claims**

1. An information processing apparatus (10; 10a; 10b) comprising:

    a behavior state detection sensor (20) configured to detect behavior state information on a behavior state of a user;
    a behavior pattern information generation unit (42) configured to generate behavior pattern information in a multidimensional space formed of coordinate axes based on the behavior state information to generate a group of spaces in each of which density of a behavior pattern information group as a collection of pieces of the behavior pattern information exceeds predetermined density, the coordinate axes representing at least parameters of a date and time, a place, and a duration of detection of the behavior state;

    **characterised in that** the information processing apparatus is further comprising:

    a behavior score calculation unit (44) configured to calculate, as a behavior score, information on a size of the space including the behavior pattern information group; and
    a behavior pattern identification unit (46) configured to identify, as a behavior pattern of the user, the behavior pattern information group in the space for which a value of the behavior score is smaller than a predetermined value.

2. The information processing apparatus (10; 10a; 10b) according to claim 1, wherein the behavior pattern identification unit (46) is configured to determine, as an extraordinary behavior, the behavior pattern for which the value of the behavior score is larger than a first threshold, and to determine, as an ordinary behavior, the behavior pattern for which the value of the behavior score is smaller than a second threshold.

3. The information processing apparatus (10; 10a; 10b) according to claim 1 or 2, wherein the behavior pattern information generation unit (42) is configured to plot and generate the behavior pattern information in the multidimensional space.

4. The information processing apparatus (10a; 10b) according to any one of claims 1 to 3, further comprising:

a biological sensor (32) configured to detect biological information related to biological information on the user; and

an activity level score calculation unit (54) configured to calculate an autonomic nerve activity level of the user based on the biological information, and to calculate an activity level score of the user based on the autonomic nerve activity level, the behavior score, and the behavior pattern.

5. The information processing apparatus (10b) according to claim 4, further comprising:
an activity level score correction unit (56) configured to correct the activity level score based on one of a country and a region in which the behavior pattern of the user is identified.

6. The information processing apparatus (10a; 10b) according to claim 4 or 5, wherein the activity level score calculation unit (54) is configured to calculate a current activity level score of the user based on a history of activity level scores of the user.

7. The information processing apparatus (10a; 10b) according to any one of claims 4 to 6, further comprising:
an output control unit (50) configured to output a temporal change of the activity level score of the user by using an output unit (24).

8. The information processing apparatus (10a; 10b) according to any one of claims 4 to 7, wherein a current activity level score of the user is calculated based on a history of a different user for whom a behavior pattern and an activity level score are similar to the user.

9. An information processing method comprising the steps of:

detecting behavior information on a behavior state of a user (S10; S20; S40; S60; S80; S120; S160);
generating behavior pattern information in a multidimensional space formed of coordinate axes based on the behavior state information to generate a group of spaces in each of which density of a behavior pattern information group as a collection of pieces of the behavior pattern information exceeds predetermined density, the coordinate axes representing at least parameters of a date and time, a place, and a duration of detection of the behavior state (S11, S12; S21, S22; S42, S43; S62, S63; S82, S83; S122, S123; S162, S163);

**characterised by** the information processing method further comprising the steps of:

calculating, as a behavior score, information on a size of the space including the behavior pattern information group (S13; S23; S44; S64; S84; S124; S164); and
identifying, as a behavior pattern of the user, the behavior pattern information group in the space for which a value of the behavior score is smaller than a predetermined value (S15; S25, S29; S46; S66; S86, S93; S126; S166).

10. A program that causes a computer to execute the steps of:

detecting behavior state information on a behavior state of a user (S10; S20; S40; S60; S80; S120; S160);
generating behavior pattern information in a multidimensional space formed of coordinate axes based on the behavior state information to generate a group of spaces in each of which density of a behavior pattern information group as a collection of pieces of the behavior pattern information exceeds predetermined density, the coordinate axes representing at least parameters of a date and time, a place, and a duration of detection of the behavior state (S11, S12; S21, S22; S42, S43; S62, S63; S82, S83; S122, S123; S162, S163);

**characterised in that** the program causes the comnputer to execute the further steps of:

calculating, as a behavior score, information on a size of the space including the behavior pattern information group (S13; S23; S44; S64; S84; S124; S164); and
identifying, as a behavior pattern of the user, the behavior pattern information group in the space for which a value of the behavior score is smaller than a predetermined value (S15; S25, S29; S46; S66; S86, S93; S126; S166).

**Patentansprüche**

1. Informationsverarbeitungsvorrichtung (10; 10a; 10b), die Folgendes aufweist:

einen Verhaltenszustandsdetektionssensor (20), der konfiguriert ist zum Detektieren eines Verhaltenszustands eines Benutzers;

eine Verhaltensmusterinformationserzeugungseinheit (42), die konfiguriert ist zum Erzeugen von Verhaltensmusterinformationen in einem mehrdimensionalen Raum, der aus Koordinatenachsen gebildet wird, die auf den Verhaltenszustandsinformationen basieren, um eine Gruppe von Räumen zu erzeugen, in denen die Dichte einer Verhaltensmusterinformationsgruppe als Zusammenstellung von Teilen der Verhaltensmusterinformation eine vorbestimmte Dichte überschreitet, wobei die Koordinatenachsen zumindest Parameter eines Datums und einer Zeit, eines Ortes und einer Dauer der Detektion eines Verhaltenszustands repräsentieren;

**dadurch gekennzeichnet, dass** die Informationsverarbeitungsvorrichtung weiter Folgendes aufweist:

eine Verhaltensbewertungs- bzw. Verhaltensscoreberechnungseinheit (44), die konfiguriert ist zum Berechnen von Informationen über eine Größe eines Raums, der die Verhaltensmusterinformationsgruppe enthält, als eine Verhaltensbewertung bzw. einen Verhaltensscore; und

eine Verhaltensmusteridentifikationseinheit (46), die konfiguriert ist zum Identifizieren der Verhaltensmusterinformationsgruppe in dem Raum, für die ein Wert des Verhaltensscores kleiner als ein vorbestimmter Wert ist, als ein Verhaltensmuster des Benutzers.

2. Informationsverarbeitungsvorrichtung (10; 10a; 10b) nach Anspruch 1, wobei die Verhaltensmusteridentifikationseinheit (46) so konfiguriert ist, dass sie das Verhaltensmuster, für das der Wert des Verhaltensscores größer als ein erster Schwellenwert ist, als außergewöhnliches Verhalten bestimmt bzw. festlegt, und das Verhaltensmuster, für das der Wert des Verhaltensscores kleiner als ein zweiter Schwellenwert ist, als gewöhnliches Verhalten bestimmt.

3. Informationsverarbeitungsvorrichtung (10; 10a; 10b) nach Anspruch 1 oder 2, wobei die Verhaltensmusterinformationserzeugungseinheit (42) so konfiguriert ist, dass sie die Verhaltensmusterinformationen in dem mehrdimensionalen Raum darstellt und generiert.

4. Informationsverarbeitungsvorrichtung (10a; 10b) nach einem der Ansprüche 1 bis 3, die weiter Folgendes aufweist:

einen Biosensor (32), der konfiguriert ist zum Detektieren von biologischen Informationen, die mit biologischen Informationen des Benutzers in Beziehung stehen; und

eine Aktivitätsniveaubewertungs- bzw. Aktivitätsniveauscoreberechnungseinheit (54), die konfiguriert ist zum Berechnen eines Aktivitätsniveaus des autonomen Nervensystems des Benutzers basierend auf den biologischen Informationen und zum Berechnen eines Aktivitätsniveauscores des Benutzers basierend auf dem Aktivitätsniveau des autonomen Nervensystems, dem Verhaltensscore und dem Verhaltensmuster.

5. Informationsverarbeitungsvorrichtung (10b) nach Anspruch 4, die weiter Folgendes aufweist:
eine Aktivitätsniveauscorekorrektureinheit (56), die konfiguriert ist zum Korrigieren des Aktivitätsniveauscores basierend auf einem Land oder einer Region, in der das Verhaltensmuster des Benutzers identifiziert wurde.

6. Informationsverarbeitungsvorrichtung (10a; 10b) nach Anspruch 4 oder 5, wobei die Aktivitätsniveauscoreberechnungseinheit (54) konfiguriert ist zum Berechnen eines aktuellen Aktivitätsniveauscores des Benutzers basierend auf einem Verlauf von Aktivitätsniveauscores des Benutzers.

7. Informationsverarbeitungsvorrichtung (10a; 10b) nach einem der Ansprüche 4 bis 6, die weiter Folgendes aufweist:
eine Ausgabesteuereinheit (50), die konfiguriert ist zum Ausgeben einer zeitlichen Änderung des Aktivitätsniveauscores des Benutzers mittels einer Ausgabeeinheit (24).

8. Informationsverarbeitungsvorrichtung (10a; 10b) nach einem der Ansprüche 4 bis 7, wobei ein aktueller Aktivitätsniveauscore des Benutzers basierend auf einem Verlauf eines anderen Benutzers berechnet wird, für den ein Verhaltensmuster und ein Aktivitätsniveauscore ähnlich wie bei dem Benutzers sind.

9. Informationsverarbeitungsverfahren, das folgende Schritte aufweist:

Detektieren von Verhaltensinformationen über den Verhaltenszustand eines Benutzers (S10; S20; S40; S60; S80; S120; S160);
Erzeugen von Verhaltensmusterinformationen in einem mehrdimensionalen Raum, der aus Koordinatenachsen gebildet wird, die auf den Verhaltenszustandsinformationen basieren, um eine Gruppe von Räumen zu erzeugen, in denen jeweils die Dichte einer Verhaltensmusterinformationsgruppe als Zusammenstellung von Teilen der Verhaltensmusterinformationen eine vorbestimmte Dichte überschreitet, wobei die Koordinatenachsen zumindest Parameter eines Datums und einer Zeit, eines Ortes und einer Dauer der Detektion des Verhaltenszustands darstellen (S11, S12; S21, S22; S42, S43; S62, S63; S82, S83; S122, S123; S162, S163);

**dadurch gekennzeichnet, dass** das Informationsverarbeitungsverfahren weiter folgende Schritte aufweist:

Berechnen von Informationen über eine Größe des Raums, der die Verhaltensmusterinformationsgruppe enthält, als Verhaltensscore (S13; S23; S44; S64; S84; S124; S164); und

Identifizieren der Verhaltensmusterinformationsgruppe in dem Raum für die der Verhaltensscore kleiner als ein vorbestimmter Wert ist als ein Verhaltensmuster des Benutzers (S15; S25, S29; S46; S66; S86, S93; S126; S166).

**10.** Programm, das einen Computer veranlasst, folgende Schritte auszuführen:

Detektieren von Verhaltenszustandsinformationen über den Verhaltenszustand eines Benutzers (S10; S20; S40; S60; S80; S120; S160);

Erzeugen von Verhaltensmusterinformationen in einem mehrdimensionalen Raum, der aus Koordinatenachsen gebildet wird, die auf den Verhaltenszustandsinformationen basieren, um eine Gruppe von Räumen zu erzeugen, in denen jeweils die Dichte einer Verhaltensmusterinformationsgruppe als Zusammenstellung von Teilen der Verhaltensmusterinformationen eine vorbestimmte Dichte überschreitet, wobei die Koordinatenachsen zumindest Parameter eines Datums und einer Zeit, eines Ortes und einer Dauer der Detektion des Verhaltenszustands darstellen (S11, S12; S21, S22; S42, S43; S62, S63; S82, S83; S122, S123; S162, S163);

**dadurch gekennzeichnet, dass** das Programm den Computer veranlasst, folgende weitere Schritte auszuführen:

Berechnen von Informationen über eine Größe des Raums, der die Verhaltensmusterinformationsgruppe enthält, als Verhaltensscore (S13; S23; S44; S64; S84; S124; S164); und

Identifizieren der Verhaltensmusterinformationsgruppe in dem Raum für die der Verhaltensscore kleiner als ein vorbestimmter Wert ist als ein Verhaltensmuster des Benutzers (S15; S25, S29; S46; S66; S86, S93; S126; S166).

**Revendications**

**1.** Appareil de traitement d'informations (10 ; 10a ; 10b) comprenant :

un capteur de détection d'état de comportement (20) configuré pour détecter des informations d'état de comportement d'un utilisateur ;

une unité de génération d'informations sur un modèle de comportement (42) configurée pour générer des informations sur un modèle de comportement dans un espace multidimensionnel constitué d'axes de coordonnées basés sur les informations sur l'état du comportement pour générer un groupe d'espaces dans chacun desquels la densité d'un groupe d'informations sur le modèle de comportement, en tant que collection d'éléments d'informations sur le modèle de comportement, dépasse une densité prédéterminée, les axes de coordonnées représentant au moins des paramètres d'une date et d'une heure, d'un lieu et d'une durée de détection de l'état du comportement ;

**caractérisé en ce que** l'appareil de traitement d'informations comprend en outre :

une unité de calcul de score de comportement (44) configurée pour calculer, en tant que score de comportement, des informations sur la taille de l'espace incluant le groupe d'informations sur le modèle de comportement ; et

une unité d'identification de modèle de comportement (46) configurée pour identifier, en tant que modèle de comportement de l'utilisateur, le groupe d'informations sur le modèle de comportement dans l'espace pour lequel une valeur du score de comportement est inférieure à une valeur prédéterminée.

**2.** Appareil de traitement d'informations (10 ; 10a ; 10b) selon la revendication 1, dans lequel l'unité d'identification du modèle de comportement (46) est configurée pour déterminer, en tant que comportement extraordinaire, le modèle de comportement pour lequel la valeur du score de comportement est supérieure à un premier seuil, et pour déterminer, en tant que comportement ordinaire, le modèle de comportement pour lequel la valeur du score de comportement est inférieure à un deuxième seuil.

**3.** Appareil de traitement d'informations (10 ; 10a ; 10b) selon la revendication 1 ou 2, dans lequel l'unité de génération d'informations sur le modèle de comportement (42) est configurée pour tracer et générer les informations sur le modèle de comportement dans l'espace multidimensionnel.

**4.** Appareil de traitement d'informations (10a ; 10b) selon l'une quelconque des revendications 1 à 3, comprenant en outre :

un capteur biologique (12) configuré pour détecter des informations biologiques relatives à des informations biologiques de l'utilisateur ; et

une unité de calcul du score de niveau d'activité (54) configurée pour calculer un niveau d'activité nerveuse autonome de l'utilisateur sur la base des informations biologiques, et pour calculer un score de niveau d'activité de l'utilisateur sur la base du niveau d'activité nerveuse autonome, du score de comportement et du modèle de comportement.

**5.** Appareil de traitement d'informations (10b) selon la revendication 4, comprenant en outre :
une unité de correction du score de niveau d'activité (56) configurée pour corriger le score de niveau d'activité sur la base d'un pays et d'une région dans lesquels le modèle de comportement de l'utilisateur est identifié.

**6.** Appareil de traitement d'informations (10a ; 10b) selon la revendication 4 ou 5, dans lequel l'unité de calcul du score de niveau d'activité (54) est configurée pour calculer un score de niveau d'activité actuel de l'utilisateur sur la base d'un historique des scores de niveau d'activité de l'utilisateur.

**7.** Appareil de traitement d'informations (10a ; 10b) selon l'une quelconque des revendications 4 à 6, comprenant en outre :
une unité de commande de sortie (50) configurée pour fournir en sortie une variation temporelle du score du niveau d'activité de l'utilisateur à l'aide d'une unité de sortie (24).

**8.** Appareil de traitement d'informations (10a ; 10b) selon l'une quelconque des revendications 4 à 7, dans lequel un score de niveau d'activité actuel de l'utilisateur est calculé sur la base d'un historique d'un utilisateur différent pour lequel un modèle de comportement et un score de niveau d'activité sont similaires à ceux de l'utilisateur.

**9.** Procédé de traitement d'informations comprenant les étapes suivantes :

la détection d'informations de comportements sur l'état de comportement d'un utilisateur (S10 ; S20 ; S40 ; S60 ; S80 ; S120 ; S160) ;
la génération d'informations sur le modèle de comportement dans un espace multidimensionnel constitué d'axes de coordonnées basés sur les informations sur l'état du comportement pour générer un groupe d'espaces dans chacun desquels la densité d'un groupe d'informations sur le modèle de comportement, en tant que collection d'éléments d'informations sur le modèle de comportement, dépasse une densité prédéterminée, les axes de coordonnées représentant au moins des paramètres d'une date et d'une heure, d'un lieu et d'une durée de détection de l'état du comportement (S11, S12 ; S21, S22 ; S42, S43 ; S62, S63 ; S82, S83 ; S122, S123 ; S162, S163) ;
**caractérisé en ce que** le procédé de traitement d'informations comprend en outre les étapes suivantes :

le calcul, en tant que score de comportement, d'informations sur la taille de l'espace incluant le groupe d'informations sur le modèle de comportement (S13 ; S23 ; S44 ; S64 ; S84 ; S124 ; S164) ; et
l'identification, en tant que modèle de comportement de l'utilisateur, du groupe d'informations sur le modèle de comportement dans l'espace pour lequel une valeur du score de comportement est inférieure à une valeur prédéterminée (S15 ; S25, S29 ; S46 ; S66 ; S806, S93 ; S126 ; S166).

**10.** programme qui amène un ordinateur à exécuter les étapes suivantes :

la détection d'informations de comportements sur l'état de comportement d'un utilisateur (S10 ; S20 ; S40 ; S60 ; S80 ; S120 ; S160) ;
la génération d'informations sur le modèle de comportement dans un espace multidimensionnel constitué d'axes de coordonnées basés sur les informations sur l'état du comportement pour générer un groupe d'espaces dans chacun desquels la densité d'un groupe d'informations sur le modèle de comportement, en tant que collection d'éléments d'informations sur le modèle de comportement, dépasse une densité prédéterminée, les axes de coordonnées représentant au moins des paramètres d'une date et d'une heure, d'un lieu et d'une durée de détection de l'état du comportement (S11, S12 ; S21, S22 ; S42, S43 ; S62, S63 ; S82, S83 ; S122, S123 ; S162, S163) ;

**caractérisé en ce que** le procédé de traitement d'informations comprend en outre les étapes suivantes :

le calcul, en tant que score de comportement, d'informations sur la taille de l'espace incluant le groupe d'informations sur le modèle de comportement (S13 ; S23 ; S44 ; S64 ; S84 ; S124 ; S164) ; et

l'identification, en tant que modèle de comportement de l'utilisateur, du groupe d'informations sur le modèle de comportement dans l'espace pour lequel une valeur du score de comportement est inférieure à une valeur prédéterminée (S15 ; S25, S29 ; S46 ; S66 ; S806, S93 ; S126 ; S166).

# FIG.1

# FIG.2

BEHAVIOR STATE SENSOR — 20

- CAMERA — 20A
- MICROPHONE — 20B
- GNSS RECEIVER — 20C
- ACCELERATION SENSOR — 20D
- GYRO SENSOR — 20E
- LIGHT SENSOR — 20F
- TEMPERATURE SENSOR — 20G
- HUMIDITY SENSOR — 20H

INPUT UNIT — 22

OUTPUT UNIT — 24

- DISPLAY UNIT — 24A
- SOUND OUTPUT UNIT — 24B

COMMUNICATION UNIT — 26

STORAGE UNIT — 28

- LEARNING MODEL — 28A
- MAP DATA — 28B

CONTROL UNIT — 30

- BEHAVIOR STATE INFORMATION ACQUISITION UNIT — 40
- BEHAVIOR STATE PATTERN INFORMATION GENERATION UNIT — 42
- BEHAVIOR SCORE CALCULATION UNIT — 44
- BEHAVIOR PATTERN IDENTIFICATION UNIT — 46
- STORAGE CONTROL UNIT — 48
- OUTPUT CONTROL UNIT — 50

— 10

# FIG.3

```
START
```

↓ S10

ACQUIRE BEHAVIOR STATE
INFORMATION

↓ S11

GENERATE BEHAVIOR PATTERN
INFORMATION

↓ S12

GENERATE GROUP OF BEHAVIOR
PATTERN INFORMATION GROUPS

↓ S13

CALCULATE BEHAVIOR SCORE

↓ S14

IS BEHAVIOR
SCORE SMALLER THAN
THRESHOLD? — NO →

↓

IDENTIFY BEHAVIOR PATTERN — S15

↓ S16

IDENTIFY TYPE OF BEHAVIOR STATE

↓ S17

STORE BEHAVIOR PATTERN

↓

IS DIFFERENT
GROUP PRESENT? — S18

↓ NO

IS PROCESS TO BE
TERMINATED? — S19 — NO →

↓ YES

```
END
```

30

# FIG.4

# FIG.5

F1

# FIG.6

```
                    ( START )
                         │
                         │               ⌐S20
         ┌──────────────────────────────────┐
         │   ACQUIRE BEHAVIOR STATE          │
         │        INFORMATION               │
         └──────────────────────────────────┘
                         │               ⌐S21
         ┌──────────────────────────────────┐
         │  GENERATE BEHAVIOR PATTERN       │
         │        INFORMATION               │
         └──────────────────────────────────┘
                         │               ⌐S22
         ┌──────────────────────────────────┐
         │  GENERATE GROUP OF BEHAVIOR      │
         │  PATTERN INFORMATION GROUPS      │
         └──────────────────────────────────┘
                         │               ⌐S23
         ┌──────────────────────────────────┐
         │    CALCULATE BEHAVIOR SCORE      │
         └──────────────────────────────────┘
                         │
                    ⌐S24 │                            ⌐S28
              IS BEHAVIOR                          IS BEHAVIOR
            SCORE SMALLER THAN    ──NO──>      SCORE EQUAL TO OR
             FIRST THRESHOLD?                  LARGER THAN FIRST
                         │                     THRESHOLD AND SMALLER
                        YES                    THAN SECOND THRESHOLD?   ──NO──┐
                         │               ⌐S25           │                     │
         ┌──────────────────────────────────┐          YES                    │
         │  IDENTIFY BEHAVIOR PATTERN OF    │           │               ⌐S29  │
         │     ORDINARY BEHAVIOR            │    ┌──────────────────────┐     │
         └──────────────────────────────────┘    │  IDENTIFY BEHAVIOR   │     │
                         │               ⌐S26    │   PATTERN OF         │     │
         ┌──────────────────────────────────┐    │ EXTRAORDINARY BEHAVIOR│    │
         │  IDENTIFY TYPE OF BEHAVIOR STATE │    └──────────────────────┘     │
         │     OF ORDINARY BEHAVIOR         │           │               ⌐S30  │
         └──────────────────────────────────┘    ┌──────────────────────┐     │
                         │               ⌐S27    │   IDENTIFY TYPE OF    │     │
         ┌──────────────────────────────────┐    │  BEHAVIOR STATE OF   │     │
         │  STORE BEHAVIOR PATTERN OF       │    │ EXTRAORDINARY BEHAVIOR│    │
         │     ORDINARY BEHAVIOR            │    └──────────────────────┘     │
         └──────────────────────────────────┘           │               ⌐S31  │
                         │                        ┌──────────────────────┐     │
                         │                        │  STORE BEHAVIOR      │     │
                         │                        │  PATTERN OF          │     │
                         │                        │ EXTRAORDINARY BEHAVIOR│    │
                         │                        └──────────────────────┘     │
                         │                                 │                    │
                         │<───────────────────────────────┴────────────────────┘
                         │               ⌐S32
    ┌──YES──        IS DIFFERENT
    │            GROUP PRESENT?
    │                    │
    │                   NO
    │                    │               ⌐S33
    │         ──NO──  IS PROCESS TO BE
    │                 TERMINATED?
    │                    │
    │                   YES
    │                    │
    │                ( END )
```

# FIG.7

# FIG.8

10a

**20**

### BEHAVIOR STATE SENSOR

20A
CAMERA

20B
MICROPHONE

20C
GNSS RECEIVER

20D
ACCELERATION SENSOR

20E
GYRO SENSOR

20F
LIGHT SENSOR

20G
TEMPERATURE SENSOR

20H
HUMIDITY SENSOR

**32**

### BIOLOGICAL SENSOR

32A
PULSE WAVE SENSOR

**22**

### INPUT UNIT

**24**

### OUTPUT UNIT

24A
DISPLAY UNIT

24B
SOUND OUTPUT UNIT

**26**

### COMMUNICATION UNIT

**28**

### STORAGE UNIT

28A
LEARNING MODEL

28B
MAP DATA

**30a**

### CONTROL UNIT

40
BEHAVIOR STATE INFORMATION ACQUISITION UNIT

42
BEHAVIOR STATE PATTERN INFORMATION GENERATION UNIT

44
BEHAVIOR SCORE CALCULATION UNIT

46
BEHAVIOR PATTERN IDENTIFICATION UNIT

48
STORAGE CONTROL UNIT

50
OUTPUT CONTROL UNIT

52
BIOLOGICAL INFORMATION ACQUISITION UNIT

54
ACTIVITY LEVEL SCORE CALCULATION UNIT

# FIG.9

```
                    ┌─────────────┐
                    │    START    │
                    └──────┬──────┘
                           │                    ⌐S40
                ┌──────────▼──────────┐
                │  ACQUIRE BEHAVIOR   │
                │  STATE INFORMATION  │
                └──────────┬──────────┘
                           │                    ⌐S41
                ┌──────────▼──────────────────┐
                │ ACQUIRE BIOLOGICAL INFORMATION │
                └──────────┬──────────────────┘
                           │                    ⌐S42
                ┌──────────▼──────────┐
                │  GENERATE BEHAVIOR  │
                │ PATTERN INFORMATION │
                └──────────┬──────────┘
                           │                    ⌐S43
                ┌──────────▼──────────────┐
                │ GENERATE GROUP OF BEHAVIOR │
                │ PATTERN INFORMATION GROUPS │
                └──────────┬──────────────┘
                           │                    ⌐S44
                ┌──────────▼──────────┐
                │ CALCULATE BEHAVIOR SCORE │
                └──────────┬──────────┘
                           │                    ⌐S45
                      ╱───────────╲         NO
                     ╱ IS BEHAVIOR ╲──────────┐
                    ╱ SCORE SMALLER ╲         │
                    ╲  THAN THRESHOLD?╱        │
                     ╲───────────────╱        │
                           │ YES              │
                           │         ⌐S46     │
                ┌──────────▼──────────┐       │
                │ IDENTIFY BEHAVIOR PATTERN │  │
                └──────────┬──────────┘       │
                           │         ⌐S47     │
                ┌──────────▼──────────────┐   │
                │ IDENTIFY TYPE OF BEHAVIOR STATE │
                └──────────┬──────────────┘   │
                           │         ⌐S48     │
                ┌──────────▼──────────────┐   │
                │ CALCULATE ACTIVITY LEVEL SCORE │
                └──────────┬──────────────┘   │
                           │         ⌐S49     │
                ┌──────────▼──────────────┐   │
                │ PROVIDE ACTIVITY LEVEL SCORE │  │
                └──────────┬──────────────┘   │
                           │         ⌐S50     │
                ┌──────────▼──────────────┐   │
                │ STORE BEHAVIOR PATTERN AND │  │
                │  ACTIVITY LEVEL SCORE     │   │
                └──────────┬──────────────┘   │
                           │◄─────────────────┘
                           │         ⌐S51
          YES         ╱─────────────╲
        ┌─────────────╱ IS DIFFERENT ╲
        │             ╲ GROUP PRESENT? ╱
        │              ╲─────────────╱
        │                    │ NO
        │                    │         ⌐S52
        │     NO        ╱──────────────╲
        │  ┌────────────╱ IS PROCESS TO BE ╲
        │  │            ╲  TERMINATED?     ╱
        │  │             ╲──────────────╱
        │  │                   │ YES
        │  │            ┌──────▼──────┐
        │  │            │     END     │
        │  │            └─────────────┘
```

# FIG.10

RR INTERVAL

INTENSITY

WR

WR

TIME

# FIG.11

F2

| D1a | D2a | D3a | D4a | D5a | D6a |
|---|---|---|---|---|---|
| | | | | | |

# FIG.12

**20**

BEHAVIOR STATE SENSOR

**20A**

CAMERA

**20B**

MICROPHONE

**20C**

GNSS RECEIVER

**20D**

ACCELERATION SENSOR

**20E**

GYRO SENSOR

**20F**

LIGHT SENSOR

**20G**

TEMPERATURE SENSOR

**20H**

HUMIDITY SENSOR

**32**

BIOLOGICAL SENSOR

**32A**

PULSE WAVE SENSOR

**22**

INPUT UNIT

**24**

OUTPUT UNIT

**24A**

DISPLAY UNIT

**24B**

SOUND OUTPUT UNIT

**26**

COMMUNICATION UNIT

**28a**

STORAGE UNIT

**28A**

LEARNING MODEL

**28B**

MAP DATA

**28C**

CORRECTION DATA

**10b**

**30b**

CONTROL UNIT

**40**

BEHAVIOR STATE INFORMATION ACQUISITION UNIT

**42**

BEHAVIOR STATE PATTERN INFORMATION GENERATION UNIT

**44**

BEHAVIOR SCORE CALCULATION UNIT

**46**

BEHAVIOR PATTERN IDENTIFICATION UNIT

**48**

STORAGE CONTROL UNIT

**50**

OUTPUT CONTROL UNIT

**52**

BIOLOGICAL INFORMATION ACQUISITION UNIT

**54**

ACTIVITY LEVEL SCORE CALCULATION UNIT

**56**

ACTIVITY LEVEL SCORE CORRECTION UNIT

# FIG.13

28C

| BEHAVIOR PATTERN | AREA A1 | AREA A2 | AREA A3 | ... |
|---|---|---|---|---|
| MP1 | 0.5 | 0.2 | 0.3 | ... |
| MP2 | 0.2 | 0.6 | 0.7 | ... |
| MP3 | 0.1 | 0.9 | 0.5 | ... |
| ... | ... | ... | ... | ... |

# FIG.14

```
                        ┌─────────────┐
                        │    START    │
                        └──────┬──────┘
                               │            ⌠S60
                    ┌──────────▼──────────────┐
                    │  ACQUIRE BEHAVIOR STATE  │
                    │       INFORMATION        │
                    └──────────┬──────────────┘
                               │            ⌠S61
                    ┌──────────▼──────────────┐
                    │ ACQUIRE BIOLOGICAL INFORMATION │
                    └──────────┬──────────────┘
                               │            ⌠S62
                    ┌──────────▼──────────────┐
                    │  GENERATE BEHAVIOR PATTERN │
                    │       INFORMATION        │
                    └──────────┬──────────────┘
                               │            ⌠S63
                    ┌──────────▼──────────────┐
                    │  GENERATE GROUP OF BEHAVIOR │
                    │ PATTERN INFORMATION GROUPS │
                    └──────────┬──────────────┘
                               │            ⌠S64
                    ┌──────────▼──────────────┐
                    │  CALCULATE BEHAVIOR SCORE │
                    └──────────┬──────────────┘
                               │            ⌠S65
                          ╱────▼────╲
                      IS BEHAVIOR            NO
                   SCORE SMALLER THAN ──────────►
                       THRESHOLD?
                          ╲────┬────╱
                            YES│       ⌠S66
                    ┌──────────▼──────────────┐
                    │  IDENTIFY BEHAVIOR PATTERN │
                    └──────────┬──────────────┘
                               │            ⌠S67
                    ┌──────────▼──────────────┐
                    │ IDENTIFY TYPE OF BEHAVIOR STATE │
                    └──────────┬──────────────┘
                               │            ⌠S68
                    ┌──────────▼──────────────┐
                    │ CALCULATE ACTIVITY LEVEL SCORE │
                    └──────────┬──────────────┘
                               │            ⌠S69
                    ┌──────────▼──────────────┐
                    │ CORRECT ACTIVITY LEVEL SCORE │
                    └──────────┬──────────────┘
                               │            ⌠S70
                    ┌──────────▼──────────────┐
                    │ PROVIDE ACTIVITY LEVEL SCORE │
                    └──────────┬──────────────┘
                               │            ⌠S71
                    ┌──────────▼──────────────┐
                    │ STORE BEHAVIOR PATTERN AND │
                    │   ACTIVITY LEVEL SCORE   │
                    └──────────┬──────────────┘
                               │            ⌠S72
                          ╱────▼────╲
          YES         IS DIFFERENT
       ◄──────────   GROUP PRESENT?
                          ╲────┬────╱
                            NO │     ⌠S73
                          ╱────▼────╲
          NO         IS PROCESS TO BE
       ◄──────────     TERMINATED?
                          ╲────┬────╱
                            YES│
                        ┌──────▼──────┐
                        │     END     │
                        └─────────────┘
```

# FIG.15

~28Ca

| AREA A1 | AREA A2 | AREA A3 | ... |
|---------|---------|---------|-----|
| 0.5 | 0.2 | 0.3 | ... |

# FIG.16

START

S80
ACQUIRE BEHAVIOR STATE INFORMATION

S81
ACQUIRE BIOLOGICAL INFORMATION

S82
GENERATE BEHAVIOR PATTERN INFORMATION

S83
GENERATE GROUP OF BEHAVIOR PATTERN INFORMATION GROUPS

S84
CALCULATE BEHAVIOR SCORE

S85
IS BEHAVIOR SCORE SMALLER THAN FIRST THRESHOLD? — NO

YES

S86
IDENTIFY BEHAVIOR PATTERN OF ORDINARY BEHAVIOR

S87
IDENTIFY TYPE OF BEHAVIOR STATE OF ORDINARY BEHAVIOR

S88
CALCULATE ACTIVITY LEVEL SCORE OF ORDINARY BEHAVIOR

S89
CORRECT ACTIVITY LEVEL SCORE OF ORDINARY BEHAVIOR

S90
PROVIDE ACTIVITY LEVEL SCORE OF ORDINARY BEHAVIOR

S91
STORE BEHAVIOR PATTERN OF ORDINARY BEHAVIOR

S92
IS BEHAVIOR SCORE EQUAL TO OR LARGER THAN FIRST THRESHOLD AND SMALLER THAN SECOND THRESHOLD? — NO

YES

S93
IDENTIFY BEHAVIOR PATTERN OF EXTRAORDINARY BEHAVIOR

S94
IDENTIFY TYPE OF BEHAVIOR STATE OF EXTRAORDINARY BEHAVIOR

S95
CALCULATE ACTIVITY LEVEL SCORE OF EXTRAORDINARY BEHAVIOR

S96
CORRECT ACTIVITY LEVEL SCORE OF EXTRAORDINARY BEHAVIOR

S97
PROVIDE ACTIVITY LEVEL SCORE OF EXTRAORDINARY BEHAVIOR

S98
STORE BEHAVIOR PATTERN OF EXTRAORDINARY BEHAVIOR AND ACTIVITY LEVEL SCORE

S99
YES — IS DIFFERENT GROUP PRESENT?

NO

S100
NO — IS PROCESS TO BE TERMINATED?

YES

END

# FIG.17

⌐10c

### 20 BEHAVIOR STATE SENSOR

- 20A CAMERA
- 20B MICROPHONE
- 20C GNSS RECEIVER
- 20D ACCELERATION SENSOR
- 20E GYRO SENSOR
- 20F LIGHT SENSOR
- 20G TEMPERATURE SENSOR
- 20H HUMIDITY SENSOR

### 32 BIOLOGICAL SENSOR

- 32A PULSE WAVE SENSOR

### 22 INPUT UNIT

### 24 OUTPUT UNIT

- 24A DISPLAY UNIT
- 24B SOUND OUTPUT UNIT

### 26 COMMUNICATION UNIT

### 28b STORAGE UNIT

- 28A LEARNING MODEL
- 28B MAP DATA
- 28D HISTORY DATA

### 30c CONTROL UNIT

- 40 BEHAVIOR STATE INFORMATION ACQUISITION UNIT
- 42 BEHAVIOR STATE PATTERN INFORMATION GENERATION UNIT
- 44 BEHAVIOR SCORE CALCULATION UNIT
- 46 BEHAVIOR PATTERN IDENTIFICATION UNIT
- 48 STORAGE CONTROL UNIT
- 50 OUTPUT CONTROL UNIT
- 52 BIOLOGICAL INFORMATION ACQUISITION UNIT
- 54 ACTIVITY LEVEL SCORE CALCULATION UNIT
- 58 HISTORY DATA ACQUISITION UNIT
- 60 LEARNING UNIT

# FIG.18A

28D$_1$

| RANK | BEHAVIOR PATTERN | BEHAVIOR SCORE | ACTIVITY LEVEL SCORE |
|---|---|---|---|
| 1 | MP4 | 10 | 99 |
| 2 | MP3 | 9 | 85 |
| 3 | MP1 | 8 | 80 |
| 4 | MP9 | 7 | 58 |
| 5 | MP3 | 7 | 53 |
| ... | ... | ... | ... |

# FIG.18B

28D$_2$

| RANK | BEHAVIOR PATTERN | BEHAVIOR SCORE | ACTIVITY LEVEL SCORE |
|---|---|---|---|
| 1 | MP4 | 8 | 90 |
| 2 | MP3 | 8 | 88 |
| 3 | MP1 | 8 | 79 |
| 4 | MP8 | 9 | 51 |
| 5 | MP5 | 9 | 49 |
| ... | ... | ... | ... |

# FIG.18C

28D₃

| RANK | BEHAVIOR PATTERN | BEHAVIOR SCORE | ACTIVITY LEVEL SCORE |
|------|------------------|----------------|----------------------|
| 1 | MP7 | 10 | 89 |
| 2 | MP2 | 6 | 71 |
| 3 | MP9 | 7 | 68 |
| 4 | MP4 | 8 | 65 |
| 5 | MP3 | 9 | 57 |
| ... | ... | ... | ... |

# FIG.19

START

↓ S110

ACQUIRE HISTORY DATA

↓ S111

PERFORM LEARNING PROCESS

↓ S112

STORE LEARNED MODEL

↓

END

# FIG.20

```
                    START

                              ⌐S120
        ACQUIRE BEHAVIOR STATE
             INFORMATION

                              ⌐S121
        ACQUIRE BIOLOGICAL INFORMATION

                              ⌐S122
        GENERATE BEHAVIOR PATTERN
             INFORMATION

                              ⌐S123
        GENERATE GROUP OF BEHAVIOR
        PATTERN INFORMATION GROUPS

                              ⌐S124
        CALCULATE BEHAVIOR SCORE

                              ⌐S125
              IS BEHAVIOR              NO
        SCORE SMALLER THAN
             THRESHOLD?

                  YES         ⌐S126
        IDENTIFY BEHAVIOR PATTERN

                              ⌐S127
        IDENTIFY TYPE OF BEHAVIOR STATE

                              ⌐S128
        CALCULATE ACTIVITY LEVEL SCORE
        BASED ON LEARNED MODEL
        CUSTOMIZED FOR USER

                              ⌐S129
        PROVIDE ACTIVITY LEVEL SCORE

                              ⌐S130
        STORE BEHAVIOR PATTERN AND
        ACTIVITY LEVEL SCORE

                              ⌐S131
   YES       IS DIFFERENT
        GROUP PRESENT?

                  NO
                              ⌐S132
   NO    IS PROCESS TO BE
        TERMINATED?

                  YES
                    END
```

# FIG.21

1

INFORMATION PROCESSING APPARATUS 10c

INFORMATION PROCESSING APPARATUS 10c

...

N

SERVER APPARATUS 100

# FIG.22

100

SERVER APPARATUS

CONTROL UNIT 120

ACQUISITION UNIT 122

DETERMINA-TION UNIT 124

REQUESTING UNIT 126

PROVIDING UNIT 128

STORAGE UNIT 130

SHARED DATA 132

N

COMMUNICA-TION UNIT 110

# FIG.23

START

↓

S140
ARE USERS WITH SIMILAR HISTORY DATA PRESENT? — NO →

↓ YES

S141
REQUEST APPROVAL FOR SHARING

↓

S142
IS REQUEST APPROVED? — NO →

↓ YES

S143
SHARE HISTORY DATA

↓

END

# FIG.24

START

↓

S150
ACQUIRE HISTORY DATA

↓

S151
ACQUIRE HISTORY DATA FROM SERVER APPARATUS

↓

S152
PERFORM LEARNING PROCESS

↓

S153
STORE LEARNED MODEL

↓

END

48

# FIG.25

```
                    ┌─────────────┐
                    │    START    │
                    └──────┬──────┘
                           │
                           ▼                      ⌐S160
        ┌──────────────────────────────────────┐
        │ ACQUIRE BEHAVIOR STATE INFORMATION    │
        └──────────────────┬───────────────────┘
                           ▼                      ⌐S161
        ┌──────────────────────────────────────┐
        │     ACQUIRE BIOLOGICAL INFORMATION    │
        └──────────────────┬───────────────────┘
                           ▼                      ⌐S162
        ┌──────────────────────────────────────┐
        │      GENERATE BEHAVIOR PATTERN        │
        │            INFORMATION                │
        └──────────────────┬───────────────────┘
                           ▼                      ⌐S163
        ┌──────────────────────────────────────┐
        │     GENERATE GROUP OF BEHAVIOR        │
        │   PATTERN INFORMATION GROUPS          │
        └──────────────────┬───────────────────┘
                           ▼                      ⌐S164
        ┌──────────────────────────────────────┐
        │       CALCULATE BEHAVIOR SCORE        │
        └──────────────────┬───────────────────┘
                           ▼                      ⌐S165
                  ◇ IS BEHAVIOR            NO
                    SCORE SMALLER THAN ─────────┐
                    THRESHOLD? ◇                │
                           │ YES                │
                           ▼           ⌐S166    │
        ┌──────────────────────────────────────┐│
        │     IDENTIFY BEHAVIOR PATTERN         ││
        └──────────────────┬───────────────────┘│
                           ▼           ⌐S167    │
        ┌──────────────────────────────────────┐│
        │   IDENTIFY TYPE OF BEHAVIOR STATE     ││
        └──────────────────┬───────────────────┘│
                           ▼           ⌐S168    │
        ┌──────────────────────────────────────┐│
        │ CALCULATE ACTIVITY LEVEL SCORE        ││
        │ BASED ON LEARNED MODEL THAT IS        ││
        │ GENERATED BY USING SHARED DATA        ││
        └──────────────────┬───────────────────┘│
                           ▼           ⌐S169    │
        ┌──────────────────────────────────────┐│
        │    PROVIDE ACTIVITY LEVEL SCORE       ││
        └──────────────────┬───────────────────┘│
                           ▼           ⌐S170    │
        ┌──────────────────────────────────────┐│
        │   STORE BEHAVIOR PATTERN AND          ││
        │      ACTIVITY LEVEL SCORE             ││
        └──────────────────┬───────────────────┘│
                           ▼◄───────────────────┘
          YES      ◇ IS DIFFERENT     ⌐S171
        ┌──────────  GROUP PRESENT? ◇
        │                  │ NO
        │                  ▼                     ⌐S172
        │    NO   ◇ IS PROCESS TO BE
        │ ┌────────  TERMINATED? ◇
        │ │                │ YES
        │ │                ▼
        │ │         ┌─────────────┐
        │ │         │     END     │
        │ │         └─────────────┘
```

# FIG.26

10d

**BEHAVIOR STATE SENSOR** — 20

CAMERA — 20A

MICROPHONE — 20B

GNSS RECEIVER — 20C

ACCELERATION SENSOR — 20D

GYRO SENSOR — 20E

LIGHT SENSOR — 20F

TEMPERATURE SENSOR — 20G

HUMIDITY SENSOR — 20H

**BIOLOGICAL SENSOR** — 32

PULSE WAVE SENSOR — 32A

**INPUT UNIT** — 22

**OUTPUT UNIT** — 24a

DISPLAY UNIT — 24A

SOUND OUTPUT UNIT — 24B

TACTILE STIMULATION OUTPUT UNIT — 24C

**COMMUNICATION UNIT** — 26

**STORAGE UNIT** — 28

LEARNING MODEL — 28A

MAP DATA — 28B

**CONTROL UNIT** — 30d

BEHAVIOR STATE INFORMATION ACQUISITION UNIT — 40

BEHAVIOR STATE PATTERN INFORMATION GENERATION UNIT — 42

BEHAVIOR SCORE CALCULATION UNIT — 44

BEHAVIOR PATTERN IDENTIFICATION UNIT — 46

STORAGE CONTROL UNIT — 48

OUTPUT CONTROL UNIT — 50

BIOLOGICAL INFORMATION ACQUISITION UNIT — 52

ACTIVITY LEVEL SCORE CALCULATION UNIT — 54

# FIG.27A

# FIG.27B

# FIG.27C

24A

r2
r1
r3
C1
C2
C3

# FIG.27D

24A

B2
B1
B3
h2
h1
h3

# FIG.27E

# FIG.28

## REFERENCES CITED IN THE DESCRIPTION

**Patent documents cited in the description**

- JP 2003046630 A
- JP 2004184351 A
- WO 2009070841 A1

**Non-patent literature cited in the description**

- **BRETT ADAMS**. Sensing and using social context. *ACM Transactions on multimedia computing communications and applications, association for computing machinery*, 20 November 2008, vol. 5 (2), 1-27
- **FUJIMOTO et al.** Normal Reference Values and Prediction Equations of Autonomic Nerve Functions Based on Variations in the R-R interval in Electrocardiographs. *J. Japan Diab. Sec.*, 1987, vol. 30 (2), 167-173
- **HAYANO et al.** Heart Rate Variability and Autonomic Nerve Functions. *SEIBUTSU BUTSURI*, 1988, vol. 28-4, 32-36